# FASCICULE DE BREVET EUROPEEN

(11) **EP 0 672 150 B1**
(45) Date de publication et mention de la délivrance du brevet: **30.05.2007**
(21) Numéro de dépôt: 93913176.9
(22) Date de dépôt: 23.06.1993
(51) Int. Cl.: C12N 15/57, C12N 1/19, C12P 21/02, C12N 9/60, C12N 15/81

(54) **LEVURES KLUYVEROMYCES MODIFIEES, PREPARATION ET UTILISATION**
VERÄNDERTE KLUVEROMYCES HEFE IHRE HERSTELLUNG UND VERWENDUNG
MODIFIED KLUYVEROMYCES YEASTS, THEIR PREPARATION AND USE

(30) Priorité: 25.06.1992 FR 9207785
(43) Date de publication de la demande: 20.09.1995
(73) Titulaire: Aventis Pharma S.A., 92160 Antony (FR)
(72) Inventeur: FLEER, Reinhard, F-91440 Bures-sur-Ivette (FR); FOURNIER, Alain, F-92000 Chatenay-Malabry (FR); YEH, Patrice, F-75005 Paris (FR)
(74) Mandataire: Becker, Philippe
(86) Numéro de dépôt international: PCT/FR1993/000623
(87) Numéro de publication internationale: WO 1994/000579

(56) Documents cités:
- EP-A- 0 240 224
- EP-A- 0 301 670
- EP-A- 0 327 797
- EP-A- 0 336 056
- EP-A- 0 390 676
- WO-A-92/17595
- CHEMICAL ABSTRACTS, vol. 99, no. 15, 10 Octobre 1983, Columbus, Ohio, US; abstract no. 120893, P. GRIEVE ET AL 'Partial characterization of cheese-ripening proteinases produced by the yeast Kluyveromyces lactis' page 520 ;
- FEBS LETTERS. vol. 234, no. 2, Juillet 1988, AMSTERDAM NL pages 464 - 470 C. TANGUE-ROUGEAU ET AL 'The Kluyveromyces lactis KEX1 gene encodes a subtilisin-type serine proteinase'
- CELL. vol. 48, 13 Mars 1987, CAMBRIDGE, NA US pages 887 - 897 L.A. VALLS ET AL 'Protein sorting in yeast ....'
- MOLECULAR AND CELLULAR BIOLOGY vol. 7, no. 12, Décembre 1987, pages 4390 - 4399 C.M. MOEHLE ET AL 'Protease B of the lysosomelike vacuale of the yeast ...'
- NUCLEIC ACIDS RESEARCH. vol. 17, no. 4, 1989, ARLINGTON, VIRGINIA US page 1779 T.J. LOTT ET AL 'Nucleotide sequence of the Candida albicans aspartyl proteinase gene'
- GENE. vol. 121, Novembre 1992, AMSTERDAM NL pages 171 - 177 M. MUKHTAR ET AL 'The carboxypeptidase Y-encoding gene from Candida albicans......'
- AYUB ET AL APPL. MICROBIOL. BIOTECHNOL. vol. 37, 1992, pages 615 - 620

## Description

La présente invention concerne de nouvelles levures appartenant au genre Kluyveromyces, génétiquement modifiées, et leur utilisation pour produire avantageusement des protéines recombinantes.

Les progrès accomplis dans le domaine de la biologie moléculaire ont permis de modifier des microorganismes pour leur faire produire des protéines d'intérêt et par exemple des protéines hétérologues (protéines de mammifères, protéines artificielles, protéines chimériques, etc). En particulier, de nombreuses études génétiques ont porté sur la bactérie Escherichia coli et la levure Saccharomyces cerevisiae. Plus récemment, des outils génétiques ont été développés afin d'utiliser la levure Kluyveromyces comme cellule hôte pour la production de protéines recombinantes. La mise en évidence du plasmide pKD1 originaire de K. drosophilarum (EP 241 435) a permis de développer un système hôte vecteur particulièrement avantageux pour la sécrétion de protéines recombinantes (EP 361 991, EP 413 622).

Toutefois, l'application de ce système de production est encore limitée, en particulier par les problèmes d'efficacité d'expression des gènes dans ces microorganismes recombinés, par les problèmes de stabilité des plasmides, et également par les problèmes de dégradation des produits recombinants par les cellules dans lesquelles ils sont synthétisés. Un phénomène de protéolyse peut en effet se manifester lors du transit de la protéine d'intérêt dans la voie sécrétoire de la levure recombinée, ou par l'existence de protéases sécrétées ou présentes dans le milieu de culture à la suite d'une lyse cellulaire non souhaitée intervenant lors de la fermentation.

La demanderesse a maintenant montré qu'il est possible d'améliorer les niveaux de production desdites protéines recombinantes, c'est-à-dire dans leur forme intégrale, dans les levures Kluyveromyces, en modifiant au moins un gène codant pour une protéase cellulaire, et notamment une protéase transitant par la voie sécrétoire. De manière surprenante, la demanderesse a de plus montré que de telles modifications sont particulièrement avantageuses puisqu'elles permettent d'augmenter les taux de production de protéines recombinantes, et ceci est d'autant plus avantageux que ladite modification est sans effet apparent sur la vitesse de croissance et la viabilité des cellules modifiées dans des conditions industrielles de fermentation. Toujours de manière surprenante, la demanderesse a également montré que lesdites modifications n'affectent pas la stabilité des levures transformées, ce qui permet d'utiliser lesdites levures de façon particulièrement avantageuse pour produire des protéines recombinantes.

La présente invention a donc pour objet des levures du genre Kluyveromyces présentant une ou plusieurs modifications génétiques d'au moins un gène codant pour une protéase, modifiant l'activité protéolytique desdites levures. Préférentiellement, la ou les modifications génétiques rendent ledit gène partiellement ou totalement incapable de coder pour la protéase naturelle. Dans un autre mode préféré de l'invention, le ou les gènes ainsi modifiés génétiquement codent pour une protéase non fonctionelle, ou pour un mutant ayant un spectre d'activité protéolytique modifié. Dans un autre mode préféré de l'invention, le ou les gènes codant pour lesdites protéases sont placés sous contrôle d'un promoteur régulé.

Les levures du genre Kluyveromyces selon l'invention comprennent les levures telles que définies par van der Walt [in : The Yeasts (1987) NJ.W. Kreger-van Rij (ed) ; Elsevier ; p. 224], et préférentiellement les levures K. marxianus var. lactis (K. lactis), K. marxianus var. marxianus (K. fragilis), K. marxianus var. drosophilarum (K. drosophilarum). K. waltii, etc...

Par modification génétique, on doit entendre plus particulièrement toute suppression, substitution, délétion, ou addition d'une ou plusieurs bases dans le ou les gènes considérés. De telles modifications peuvent être obtenues in vitro (sur de l'ADN isolé) ou in situ, par exemple, au moyens des techniques du génie génétique, ou encore en exposant lesdites levures à un traitement au moyen d'agents mutagènes. Par agents mutagènes, on peut citer par exemple les agents physiques tels que les rayonnements énergétiques (rayons X, g, ultra violet, etc..), ou les agents chimiques capables de réagir avec différents groupements fonctionnels des bases de l'ADN, et par exemple les agents alkylants [éthylméthane sulfonate (EMS), N-méthyl-N'-nitro-N-nitrosoguanidine, N-nitroquinoléine-1-oxyde (NQO)], les agents bialkylants, les agents intercalants, etc... Par délétion on entend toute suppression du gène considéré. Il peut s'agir notamment d'une partie de la région codant pour lesdites protéases, et/ou de tout ou partie de la région promotrice de la transcription.

Les modifications génétiques peuvent également être obtenues par disruption génique, par exemple selon le protocole initialement décrit par Rothstein [Meth. Enzymol. 101 (1983) 202]. Dans ce cas, l'intégralité de la séquence codante sera préférentiellement perturbée pour permettre le remplacement, par recombinaison homologue, de la séquence génomique sauvage par une séquence non fonctionnelle ou mutante.

La ou lesdites modifications génétiques peuvent être localisées dans le gène codant pour lesdites protéases, ou en dehors de la région codant pour lesdites protéases, et par exemple dans les régions responsables de l'expression et/ou de la régulation transcriptionelle desdits gènes. L'incapacité desdits gènes à coder pour les protéases naturelles peut se manifester soit par la production d'une protéine inactive en raison de modifications structurales ou conformationelles, soit par l'absence de production, soit par la production d'une protéase ayant une activité enzymatique altérée, ou encore par la production de la protéase naturelle à un niveau atténué ou selon un mode de régulation désiré.

Par ailleurs, certaines altérations telles que des mutations ponctuelles sont par nature capables d'être corrigées ou atténuées par des mécanismes cellulaires, par exemple lors de la réplication de l'ADN précédant la division cellulaire. De telles altérations génétiques ont alors un intérêt limité au niveau industriel puisque les propriétés phénotypiques qui en résultent ne sont pas parfaitement stables. La demanderesse a maintenant mis au point un procédé permettant de préparer des levures Kluyveromyces présentant une ou plusieurs modifications génétiques d'au moins un gène codant pour une protéase, la ou lesdites modifications étant stables ségrégationellement et/ou non-réversibles. Les levures présentant de telles modifications sont particulièrement avantageuses comme hôte cellulaire pour la production de protéines recombinantes. L'invention permet également la réalisation de levures dans lesquelles la ou les modifications apportées rendent le ou les gènes considérés totalement ou seulement partiellement incapables de produire une protéase fonctionnelle.

Préférentiellement, les levures selon l'invention présentent une ou plusieurs modifications génétiques stables ségrégationellement. Toujours selon un mode préféré, la ou les modifications génétiques son non-réversibles. Encore selon un mode préféré de l'invention, la ou les modifications génétiques ne laissent aucune activité résiduelle au gène considéré.

Préférentiellement, le ou les gènes codant pour une ou des protéases sont choisis parmi les gènes codant pour des protéases transitant par la voie sécrétoire de Kluyveromyces. De telles protéases peuvent être localisées dans le réticulum endoplasmique, le compartiment de l'appareil de Golgi, le compartiment post-Golgi, et par exemple les vacuoles cellulaires, les vésicules de l'endosome, les vésicules de sécrétion, ou le milieu extracellulaire.

A titre d'exemple de tels gènes, on peut citer les gènes de Kluyveromyces codant pour une protéase choisie parmi les familles de la protéase A, de la protéase B, ou d'une carboxypeptidase (et par exemple la carboxypeptidase Y ou la carboxypeptidase S), ou encore l'endopeptidase KEX1 de K. lactis ou une protéase d'activité similaire, et par exemple la protéase YAP3 [Egel-Mitani et al., Yeast 6 (1990) 127], ou plus généralement toute autre protéase intervenant dans la maturation de certaines protéines sécrétées.

Dans un mode préféré de l'invention, le ou les gènes considérés codent pour des protéases n'intervenant pas dans le clivage du peptide signal des protéines recombinantes exprimées sous forme de préprotéines. On peut citer à titre d'exemples de gènes particulièrement intéressants les gènes de la protéases A, de la protéase B, et de la carboxypeptidase Y de Kluyveromyces, dont le clonage est décrit dans les exemples.

Dans un autre mode de l'invention, la ou lesdites protéases possèdent une activité de signal peptidases et la ou lesdites modifications génétiques permettent leur surexpression, ce qui est particulièrement avantageux dans le cas ou cette étape est une étape limitante de la voie sécrétoire.

L'invention a également pour objet toute levure Kluyveromyces telle que définie précédemment dans laquelle une séquence d'ADN exogène comprenant un ou plusieurs gènes codant pour une protéine d'intérêt que l'on souhaite exprimer et/ou sécréter dans ladite levure, a été introduite.

Au sens de la présente invention, on entend par séquence d'ADN exogène toute séquence d'ADN introduite artificiellement dans la levure et codant pour une ou plusieurs protéines d'intérêt. En particulier il peut s'agir de séquences d'ADN complémentaire (ADNc), de séquences artificielles ou hybrides, ou encore de séquences synthétiques ou semi-synthétiques, incluses dans une cassette d'expression permettant la synthèse dans lesdites levures de ou desdites protéines d'intérêt. Par exemple, cette séquence d'ADN exogène peut inclure une région de démarrage de la transcription, régulée ou non chez Kluyveromyces, de façon à diriger, quand cela est souhaitable, l'expression desdites protéines d'intérêt.

Préférentiellement, la séquence d'ADN exogène est incluse dans un vecteur, qui peut être soit à réplication autonome dans la levure considérée, soit de type intégratif. Plus particulièrement, des vecteurs à réplication autonome peuvent être préparés à partir de séquences à réplication autonome chez Kluyveromyces, et par exemple il peut s'agir du plasmide pKD1 [Falcone et al., Plasmids 15 (1986) 248 ; Chen et al., Nucl. Acids Res. 14 (1986) 4471] caractérisé par une haute stabilité ségrégationelle, notamment chez les différentes variétés de K. marxianus, ou du plasmide pKW1 isolé chez K. waltii [Chen et al., J. General Microbiol. 138 (1992) 337]. Des vecteurs à réplication autonome peuvent également être préparés à partir de séquences chromosomiques (ARS). S'agissant des vecteurs de type intégratifs, ceux-ci peuvent être préparés à partir de séquences chromosomiques homologues à ladite levure hôte, de façon à encadrer la séquence génétique codant pour lesdites protéines d'intérêt, et un marqueur de sélection génétique, de façon à orienter l'intégration de l'ensemble par recombinaison homologue. Dans un mode de réalisation particulier lesdites séquences homologues correspondent à des séquences génétiques provenant de la région codante de ladite protéase, ce qui permet de remplacer par recombinaison homologue la séquence originelle de ladite protéase par le marqueur de sélection et la séquence d'ADN exogène, tout en permettant la disruption génique de ladite protéase. Dans un autre mode de réalisation, la cassette d'expression est intégrée au locus codant pour les ARN ribosornaux (rDNA), permettant l'amplification génique de ladite cassette d'expression [Bergkamp et al., Curr. Genet. 21 (1992) 365]. Encore dans un autre mode de réalisation, la séquence d'ADN exogène est intégrée dans le chromosome desdites levures hôtes par recombinaison non homologue.

La séquence d'ADN exogène peut être introduite dans la levure par les techniques de l'homme de l'art, et par exemple les techniques de l'ADN recombinant, les croisements génétiques, la fusion de protoplastes, etc. Dans un mode de réalisation particulier, la séquence d'ADN exogène est introduite chez les levures Kluyveromyces par transformation, électroporation, conjugaison, ou toute autre technique décrite dans la littérature. S'agissant de transformation des levures Kluyveromyces, on peut utiliser la technique décrite par Ito et al. [J. Bacteriol. 153 (1983) 163]. La technique de transformation décrite par Durrens et al. [Curr. Genet. 18 (1990) 7] utilisant l'éthylène glycol et le diméthylsulfoxyde est également efficace. Il est aussi possible de transformer les levures par électroporation, selon la méthode décrite par Karube et al. [FEBS Letters 182 (1985) 90]. Un protocole alternatif est également décrit dans la demande de brevet EP 361991.

Lesdites levures Kluyveromyces modifiées pour leur contenu en protéases par les techniques décrites ci-avant sont avantageusement utilisées comme cellules hôtes pour produire des protéines recombinantes, et par exemple des protéines hétérologues d'intérêt pharmaceutique ou agroalimentaire. Lesdites levures hôtes sont particulièrement avantageuses puisqu'elles permettent d'augmenter la qualité et la quantité des protéines recombinantes que l'on désire produire et/ou sécréter, et que lesdites modifications génétiques desdites cellules n'affectent pas la stabilité génétique et mitotique des vecteurs d'expression desdites protéines recombinantes. Un autre objet de l'invention réside donc dans un procédé de production de protéines recombinantes selon lequel on cultive une levure telle que définie ci-dessus dans des conditions d'expression de la ou des protéines codées par la séquence d'ADN exogène, et que l'on récupère la ou les protéines d'intérêt. Dans un mode préféré, lesdites protéines d'intérêt sont sécrétées dans le milieu de culture. A titre d'exemple on peut citer des protéines existant naturellement dans la nature, ou des protéines artificielles, et par exemple des protéines hybrides. Dans ce cas, l'utilisation de cellules de levures ayant un contenu modifié en protéases est particulièrement avantageux du fait de l'exposition de la région charnière entre les différents domaines protéiques de la chimère. Dans un mode de réalisation particulier, ladite protéine artificielle comporte un peptide fusionné à une des extrémités de la chimère et est particulièrement sensible, par exemple lors du transit dans la voie sécrétoire, à une dégradation protéolytique par une exoprotéase, N- ou C-terminale, et par exemple une carboxypeptidase. Il est entendu que la dégradation protéolytique de la protéine d'intérêt peut également résulter de toute protéase cellulaire, et par exemple cytoplasmique, libérée dans le milieu extérieur du fait d'une lyse cellulaire non souhaitée pendant le procédé de la fermentation desdites levures recombinées. L'altération génétique de la séquence nucléotidique codant pour de telles protéases peut donc également résulter en un procédé particulièrement avantageux pour produire lesdites protéines d'intérêt et est également revendiquée.

Préférentiellement, le procédé selon l'invention permet la production de protéines d'intérêt pharmaceutique ou agro-alimentaire. A titre d'exemple, on peut citer les enzymes (telles que notamment la superoxide dismutase, la catalase, les amylases, les lipases, les amidases, la chymosine, etc, ou tout fragment ou dérivé de celles-ci), les dérivés sanguins (tels que la sérum-albumine, l'alpha ou la béta globine, les facteurs de la coagulation, et par exemple le facteur VIII, le facteur IX, le facteur de von Willebrand, la fibronectine, l'alpha-1 antitrypsine, etc, ou tout fragment ou dérivé de ceux-ci), l'insuline et ses variants, les lymphokines [telles que les interleukines, les interférons, les facteurs de stimulation des colonies (G-CSF, GM-CSF, M-CSF etc...), le TNF, etc, ou tout fragment ou dérivé de ceux-ci], les facteurs de croissance (tels que l'hormone de croissance, l'érythropoiétine, le FGF, l'EGF, le PDGF, le TGF, etc, ou tout fragment ou dérivé de ceux-ci), les apolipoprotéines et leurs variants moléculaires, des polypeptides antigéniques pour la réalisation de vaccins (hépatites, cytomégalovirus, virus d'Epstein-Barr, virus de l'herpès, etc), ou encore des fusions de polypeptides telles que notamment des fusions comportant une partie biologiquement active fusionnée à une partie stabilisatrice.

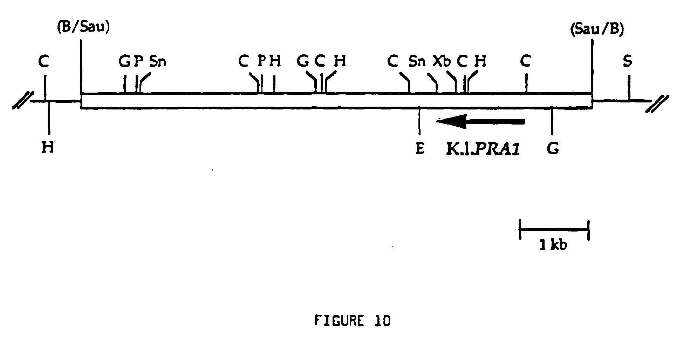

Un autre objet de la présente invention réside dans un fragment d'ADN de Kluyveromyces codant pour une protéase. La demanderesse a en effet mis en évidence, isolé et caractérisé certaines protéases de Kluyveromyces et notamment des protéases transitant par la voie sécrétoire. Plus préférentiellement, un des objets de l'invention concerne une protéase de Kluyveromyces choisie notamment parmi les protéases A, B, la carboxypeptidase Y, ainsi que la famille des sérine protéases de type subtilisine et dont un représentant est la protéase KEX1 de K. lactis [Wésolowski-Louvel et al., Yeast 4 (1988) 71]. A titre d'exemple, les séquences nucléotidiques des gènes de K. lactis codant pour les protéases A, B et la carboxypeptidase Y ont été déterminées par la demanderesse et sont présentées SEQ ID n° 3, 1 et 2 respectivement. Une carte de restriction d'un fragment chromosomique codant pour la protéase A de K. lactis est également reportée à la Figure 10. Il est entendu que tout variant génétique de ces gènes de protéases, et leur utilisation avantageuse pour produire des protéines d'intérêt, font également partie de l'invention. Lesdites variations peuvent être d'origine naturelle, mais peuvent aussi être obtenues in situ ou in vitro par les techniques du génie génétique, ou après traitement des cellules par un agent mutagène, et incluent notamment les mutations ponctuelles ou multiples, les délétions, les additions, les insertions, les protéases hybrides etc. Dans un mode de réalisation encore plus particulier, les variations génétiques peuvent également intéresser les régions de contrôle de l'expression desdites protéases, par exemple en vue d'altérer leur niveaux d'expression ou leur mode de régulation.

L'invention a également pour objet toute protéine résultant de l'expression d'un fragment d'ADN exogène tel que défini plus avant.

L'invention a également pour objet un procédé de préparation d'une levure Kluyveromyces génétiquement modifiée et son utilisation avantageuse pour produire des protéines d'intérêt. Préférentiellement, le procédé de l'invention consiste à remplacer le ou les gènes chromosomiques considérés par une version modifiée in vitro.

La présente invention sera plus complètement décrite à l'aide des exemples qui suivent, qui doivent être considérés comme illustratifs et non limitatifs.

### LEGENDE DES FIGURES

Les représentations des plasmides indiquées dans les Figures suivantes sont traçées à une échelle approximative et seuls les sites de restriction importants pour la compréhension des clonages réalisés sont indiqués.

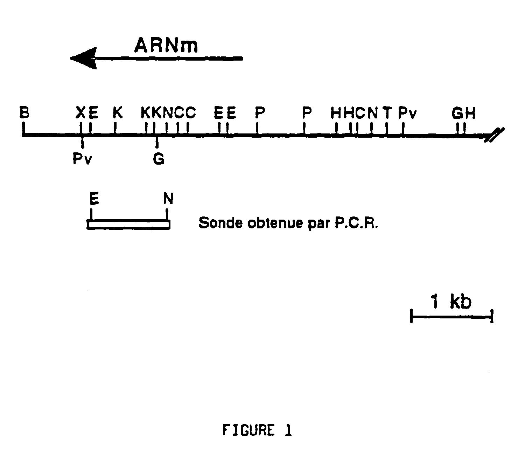

**Figure 1** : Carte de restriction de l'insert génomique du plasmide pFP8. La position des sites de coupure des endonucléases suivantes est indiquée : B=BamHI ; G=BglII ; C=ClaI ; E=EcoRI ; H=HindIII ; K=KpI ; N=NcoI ; P=PstI ; Pv=PvuII ; T=SsI ; X=XhoI. Le fragment obtenu après amplification PCR est représenté ainsi que l'ARNm du gène PRB1 de S. cerevisiae.

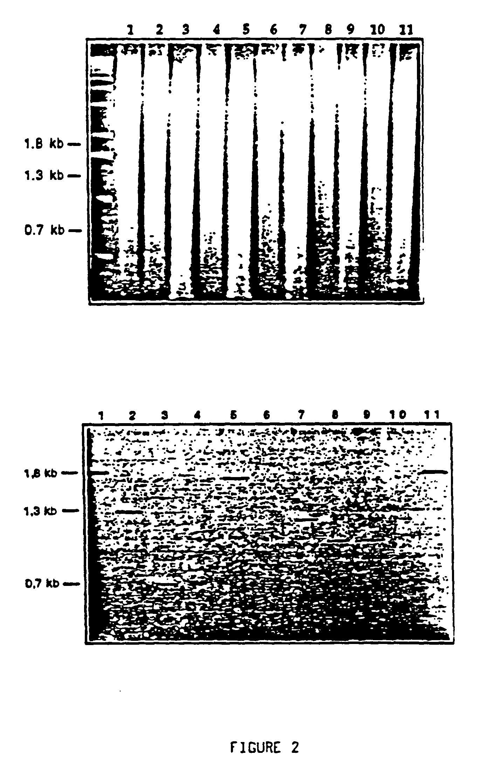

Figure 2 : Hybridation de la sonde PRB1 de S. cerevisiae avec des fragments de restriction de l'ADN génomique de K. lactis. Panneau supérieur: photo du gel d'agarose (1 %) coloré au bromure d'éthidium et avant le transfert sur le filtre de nylon; panneau inférieur: représentation schématique des signaux obtenus après hybridation du filtre avec la sonde radioactive. La numérotation correspond aux digestions suivantes : 1=EcoRI ; 2=BglII ; 3=BglII+EcoRI ; 4=HindIII ; 5=HindIII+EcoRI ; 6=PstI ; 7=PstI+ECoRI ; 8=SalI ; 9=SalI+EcoRI ; 10=BamHI ; 11=BamHI+EcoRI.

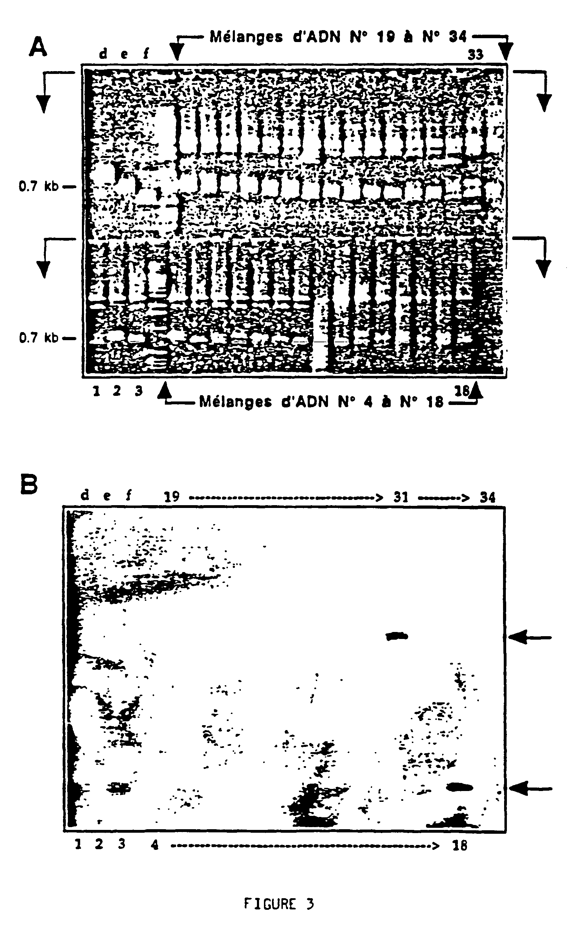

**Figure 3** : Hybridation de la sonde PRB1 de S. cerevisiae avec les 34 mini-préparations d'ADN (mélange de 10 clones chacun) après restriction double par les enzymes EcoRI et BglII. Panneau A : photo du gel d'agarose (1 %) coloré au bromure d'éthidium et avant le transfert sur le filtre de nylon ; panneau B : autoradiographie du filtre de nylon après hybridation avec la sonde PRB1 de S. cerevisiae; les sous-fractions "d", "e" et "f" correspondant à un aliquot des fragments d'ADN génomique de K. lactis après digestion totale par EcoRI+BglII et fractionnement en taille par électroélution sont indiquées.

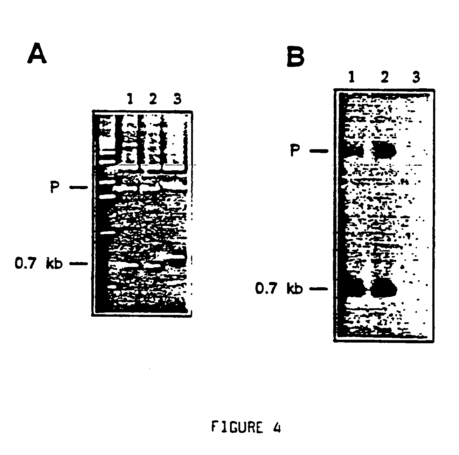

**Figure 4 :** Hybridation contrôle des clones positifs 18-3 et 31-I. Panneau A : Photo après migation sur gel d'agarose à 1 % des digestions EcoRI+BglII de l'ADN des clones 31-I (puits n° 1), 18-3 (puits n° 2) et 31-K (témoin négatif, puits n° 3). Panneau B : Autoradiographie du filtre de nylon correspondant au gel précédent après hybridation avec la sonde PRB1 de S. cerevisiae. P correspond à la localisation du plasmide (pYG1224) non digéré; la position du fragment de restriction BglII-EcoRI d'environ 0,7 kb hybridant avec la sonde radioactive est indiqué.

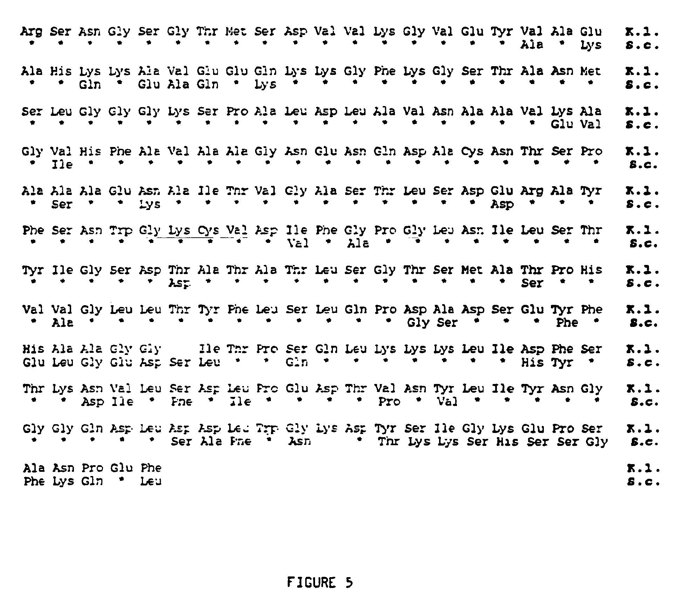

**Figure 5 :** Comparaison de la séquence protéique du fragment BglII-EcoRI d'ADN génomique de K. lactis (résidus Arg³⁰⁸ à Phe⁵³¹ de la séquence peptidique SEQ ID n° 1) avec la partie correspondante du gène PRB1 de S. cerevisiae (résidus Arg¹⁰⁵ à Leu³²⁸). Les astérisques indiquent les acides aminés conservés entre les deux séquences.

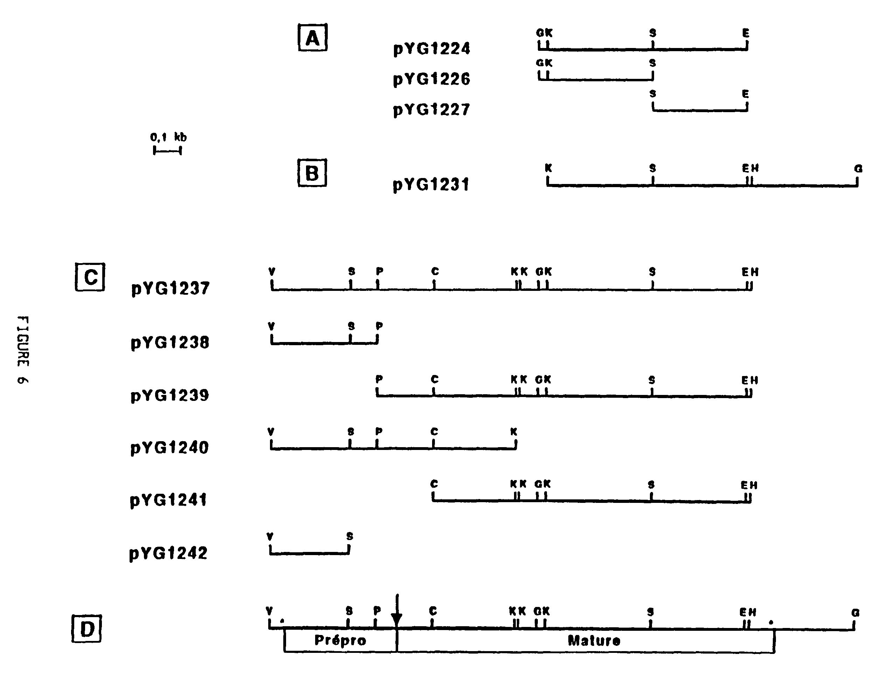

**Figure 6** : Cartes de restriction des inserts génomiques des plasmides pYG1224, pYG1226 et pYG1227 (panneau A) ; pYG1231 (panneau B) ; pYG1237, pYG1238, pYG1239, pYG1240, pYG1241 et pYG1242 (panneau C). La position des sites de coupure des endonucléases suivantes est indiquée : G=BglII; C=ClaI ; S=SalI ; E=EcoRI ; H=HindIII ; K=KpnI ; P=PstI ; V=EcoRV. Panneau D: localisation de la phase codante du gène PRB1 de K. lactis; la flèche verticale indique la position approximative de l'extrémité N-terminale de la protéine mature. La position du codon présumé d'initiation de la traduction et la position du codon spécifiant la fin de la traduction sont indiquées par une astérisque.
   **SEQ ID n° 1** : Séquence du gène PRB1 de K. lactis. Les flèches noires indiquent la position approximative de la fin des régions "pré" et "pro" de la protéase B. Les sites de restriction BglII, SalI, EcoRI et HindIII sont soulignés, ainsi que la séquence correspondant à l'oligodéoxynucléotide Sq2101.

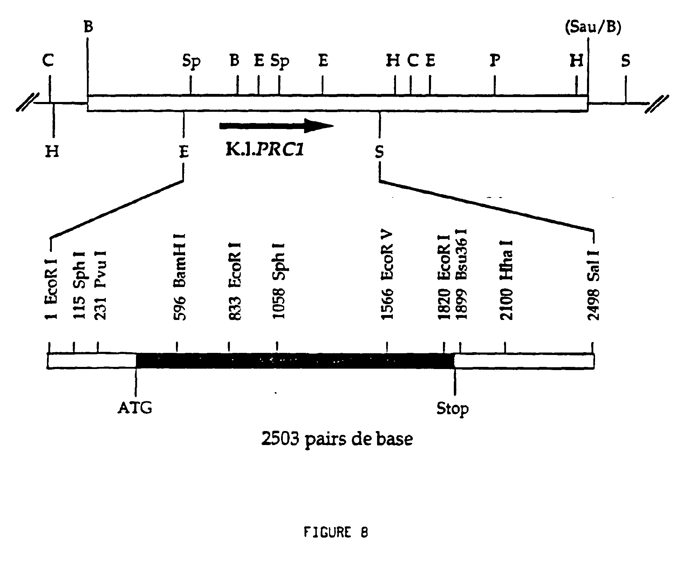

**Figure 8** : Carte de restriction de l'insert du plasmide pC34. La boîte correspond à l'insert génomique de K. lactis et la ligne correspond aux séquences du vecteur KEp6. La flèche indique la position du gène PRC1 de K. lactis. La partie détaillée comprise entre les sites EcoRI et SalI correspond à la région séquencée présentée à la Figure 9. Liste des abbréviations : C=ClaI ; H=HindIII ; B=BamHI ; E=EcoRI ; P=PstI ; S=SalI ; Sp=SphI ; Sau=Sau3A.
   **SEQ ID n° 2** : Séquence nucléotidique du fragment EcoRI-SalI incluant le gène PRC1 de K. lactis.
**Figure 10 :** Carte de restriction de l'insert du plasmide pA25/1. La boîte correspond à l'insert génomique de K. lactis et la ligne correspond aux séquences du vecteur KEp6. La flèche indique la position approximative du gène PRA1 telle qu'indiquée par une hybridation en Southern blot au moyen de sondes 3' et 5' spécifiques. Liste des abbréviations : C=ClaI ; H=HindIII ; B=BamHI ; Sau=Sau3A ; S=SalI ; P=PstI ; G=BglII ; Xb=XbaI ; Sn=SnaBI ; E=EcoRI.

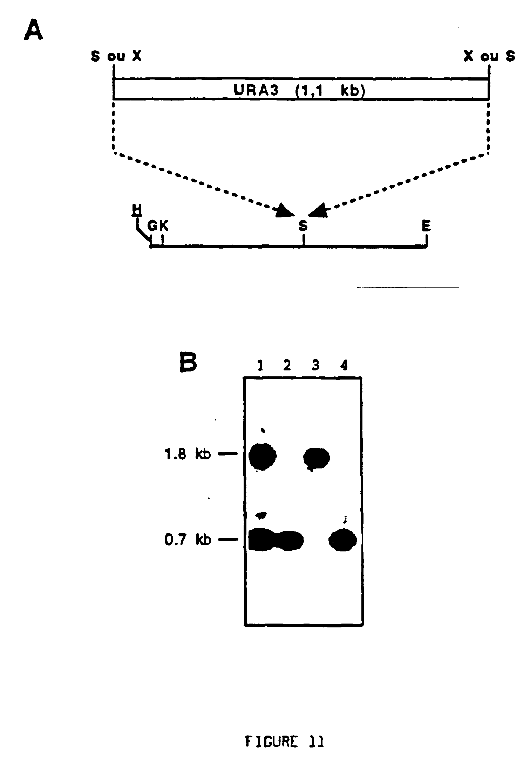

**Figure 11** : Panneau A : Carte de restriction du fragment de restriction HindIII-EcoRI du plasmide pYG1232. La position des sites de coupure des endonucléases suivantes est indiquée : G=BglII ; S=SalI ; E=EcoRI ; H=HindIII ; K=KpnI ; X=XhoI ; le site de clonage HindIII provient du vecteur et est souligné. Panneau B : Disruption du gène PRB1 de K. lactis par le marqueur de sélection URA3 de S. cerevisiae. Ce Southern blot correspond à de l'ADN génomique de K. lactis CBS 294.91 (uraA) après transformation par le fragment BglII-EcoRI du panneau A et sélection en l'absence d'uracile. Puits 1 à 3 : ADN génomique de trois transformants après restriction double BglII + EcoRI ; la souche du puits 3 est K. lactis Y750 ; puits 4 : ADN génomique de la souche CBS 294.91 après restriction double BgIII + EcoRI. La sonde radioactive utilisée correspond au fragment BglII-EcoRI du plasmide pYG1224 (Fig. 6A).

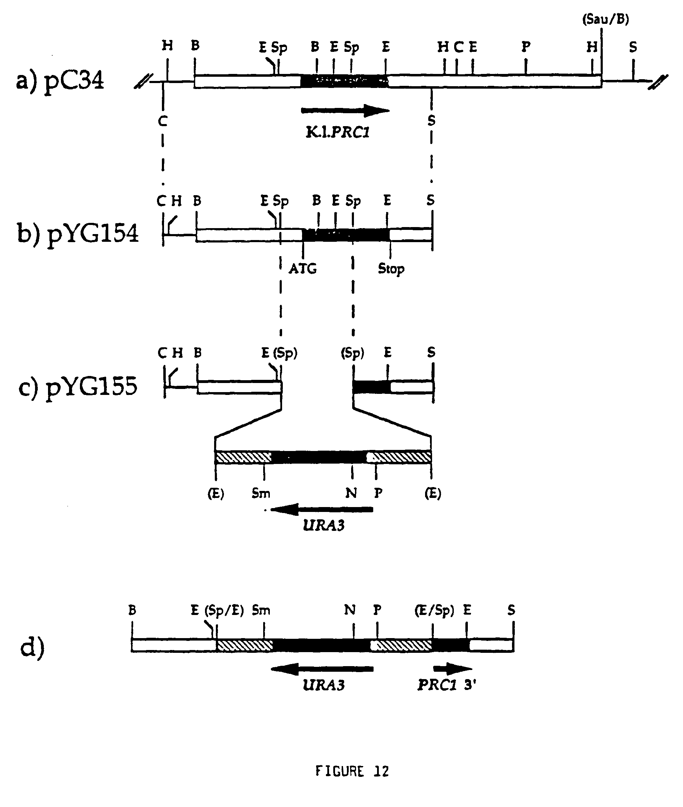

**Figure 12** : Carte de restriction des inserts des plasmides pC34 [vecteur KEp6 ; panneau a)], pYG154 [vecteur pIC-20R ; panneau b)] et pYG155 [vecteur pIC-20R ; panneau c)]. Panneau d) : Fragment utilisé pour la disruption. Les sites de restriction entre parenthèses ont été détruits par l'enzyme Klenow ou par l'ADN polymérase de T4 comme indiqué dans le texte. La boîte correspond à l'insert génomique de K. lactis et la ligne correspond aux séquences des vecteurs. Liste des abbréviations : C=ClaI ; H=HindIII ; B=BamHI ; E=EcoRI ; P=PstI ; S=SalI, Sp=SphI ; Sau=Sau3A ; Sm=SmaI ; N=NcoI.

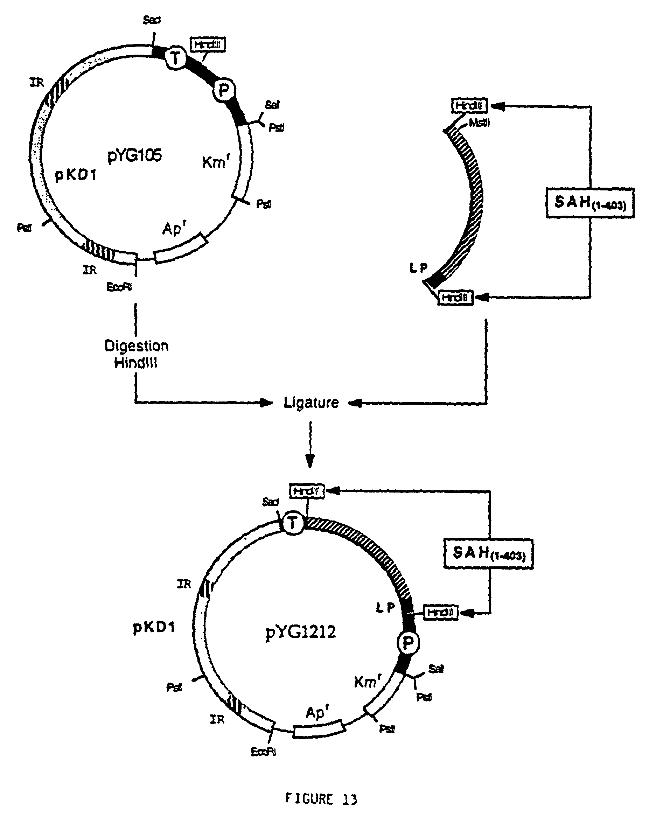

**Figure 13 :** Carte de restriction du plasmide pYG105 et stratégie de construction du plasmide pYG1212. Abbréviations utilisées : P, promoteur LAC4 de K. lactis ; T, terminateur transcriptionnel ; IR, séquences répétées inversées du plasmide pKD1 ; LP, région prépro de la SAH ; Ap^{r} et Km^{r} désignent respectivement les gènes de résistance à l'ampicilline (E. coli) et au G418 (Kluyveromyces).

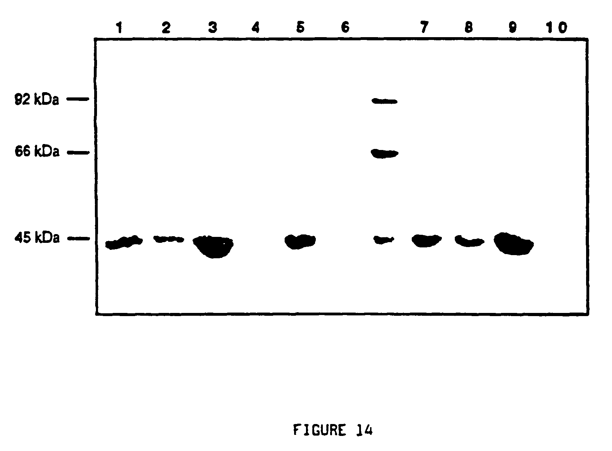

**Figure 14 :** Comparaison des capacités de sécrétion d'un variant tronqué de l'albumine humaine dans les souches K. lactis CBS 293.91 (puits 2, 4, 6, 8 et 10) ou son mutant disrupté pour le gène de la protéase B (souche Y750 ; puits 1, 3, 5, 7 et 9), après transformation par le plasmide pYG1212. Les cellules transformées sont cultivées en erlenmeyers en présence de G418 (200 mg/l) pendant 2 jours (puits 1, 2, 7 et 8), 4 jours (puits 3, 4, 9 et 10), ou 7 jours (puits 5 et 6); les puits 1 à 6 correspondent à une croissance en milieu YPD, et les puits 7 à 10 correspondent à une croissance en milieu YPL. Les dépôts sont équivalents à 50 ml de surnageants de culture.
   **SEQ ID n° 3 :** Séquence nucléotidique du fragment ClaI-EcoRI incluant le gène PRA1 de K. lactis.

### TECHNIQUES GENERALES DE CLONAGE.

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extraction de protéines au phénol ou au phénol-chloroforme, la précipitation d'ADN en milieu salin par de l'éthanol ou de l'isopropanol, la transformation dans Escherichia coli, etc..., sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982 ; Ausubel F.M. et al. (eds), "Current Protocols in Molecular Biology", John Wiley & Sons, New York, 1987].

Les enzymes de restriction ont été fournies par New England Biolabs (Biolabs), Bethesda Research Laboratories (BRL) ou Amersham et sont utilisées selon les recommandations des fournisseurs.

Les plasmides de type pBR322, pUC et les phages de la série M13 sont d'origine commerciale (Bethesda Research Laboratories). Les plasmides de type pIC ont été décrits par Marsh et al. [Gène 32 (1984) 481].

Pour les ligatures, les fragments d'ADN sont séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Biolabs) selon les recommandations du fournisseur.

Le remplissage des extrémités 5' proéminentes est effectué par le fragment de Klenow de l'ADN Polymérase I d'E. coli (Biolabs) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1. L'exonucléase Ba131 est utilisée selon les recommandations du fournisseur (Biolabs).

Les oligodéoxynucléotides sont synthétisés chimiquement selon la méthode des phosphoramidites en utilisant des groupements protecteurs B-cyanoéthyles [Sinha et al., Nucleic Acids Res. 12 (1984) 4539]. Après synthèse, les groupements protecteurs sont éliminés par traitement à l'ammoniaque et deux précipitations au butanol permettent de purifier et de concentrer les oligodéoxynucléotides [Sawadogo et Van Dyke, Nucleic Acids Res. 19 (1991) 674]. La concentration en ADN est déterminée par mesure de la densité optique à 260 nm.

La mutagénèse dirigée in vitro par oligodéoxynucléotides synthétiques est effectuée selon la méthode développée par Taylor et al. [Nucleic Acids Res. 13 (1985) 8749] en utilisant le kit distribué par Amersham.

Le fragment d'ADN utilisé pour servir de sonde moléculaire sur de l'ADN génomique de K. lactis est amplifié in vitro par la technique PCR [Polymerase-catalyzed Chain Reaction, Saiki R.K. et al., Science 230 (1985) 1350 ; Mullis K.B. et Faloona F.A., Meth. Enzym. 155 (1987) 335] sur de l'ADN de S. cerevisiae. L'amplification est automatisée (40 cycles d'amplification) et s'effectue dans un appareil Perkin Elmer Cetus (DNA thermal cycler) en utilisant La Taq polymérase (isolée de l'archaebactérie Thermophilus aquaticus) fournie par la société Perkin Elmer. Chaque cycle d'amplification comporte trois étapes :
1) Une étape de dénaturation de l'ADN à 91°C ;
2) Une étape d'hybridation des amorces d'oligodéoxynucléotides sur l'ADN matrice. La température d'hybridation est choisie cinq à dix degrés en dessous de la température de fusion des oligodéoxynucléotides (T_{1/2}). Pour des oligodéoxynucleotides d'une taille d'environ 20 mer, T_{1/2} = 2x(A+T)+4x(C+G) [Itakura et al., Ann. Rev. Biochem. 53 (1984) 323].
3) Une étape de synthèse de l'ADN complémentaire par la Taq polymérase à 72°C.

La préparation des sondes nucléotidiques radioactives est faite par incorporation d'adCTP radioactif (phosphore 32) le long de la molécule néosynthétisée à partir de 20 ng d'ADN en utilisant le kit "Random Primed DNA Labeling" commercialisé par la firme Boehringer.

Les transferts d'ADN sur membrane de nylon (Biodyne, Pall, St Germain en Laye) ou de nitrocellulose (Schleicher & Schuell, Dassel) s'effectuent selon la méthode développée initialement par Southern [J. Mol. Biol. 98 (1979) 503]. Les conditions d'hybridation et de lavage utilisées dépendent de la nature de la sonde utilisée : en conditions hétérologues (ADN génomique de K. lactis hybridé avec une sonde provenant de S.cerevisiae par exemple), l'hybridation et les lavages s'effectuent en conditions peu stringentes (hybridation pendant 15 heures à 40°C sans formamide en 5X SSC/5X Denhart, le filtre est ensuite lavé 3 fois en 5X SSC/SDS 1 % à 40°C pendant 15 minutes, puis 1 fois en 0,25X SSC/SDS 1 % pendant 10 minutes); en conditions homologues (ADN génomique de K. lactis hybridé avec une sonde provenant de K. lactis par exemple), l'hybridation et les lavages s'effectuent en conditions plus stringentes (hybridation pendant 15 heures à 40°C en 5X SSC/5X Denhart/50 % formamide, le filtre est ensuite lavé 3 fois en 5X SSC/SDS 1 % à 40°C pendant 15 minutes, puis 1 fois en 0,2X SSC/SDS 1 % pendant 10 minutes).

La vérification des séquences nucléotidiques est effectuée sur ADN plasmidique avec le kit "Sequenase version 2.0" de la société United States Biochemical Corporation, selon la méthode de Tabor et Richardson [Proc. Natl. Acad. Sci. USA 84 (1987) 4767]. Cette technique est une modification de la méthode initialement décrite par Sanger et al. [Proc. Natl. Acad. Sci. USA 74 (1977) 5463].

Les transformations de K. lactis avec l'ADN des plasmides d'expression des protéines de la présente invention sont effectuées par toute technique connue de l'homme de l'art, et dont un exemple est donné dans le texte.

Sauf indication contraire, les souches bactériennes utilisées sont E. coli MC1060 (lacIPOZYA, X74, galU, galK, strA^{r}), E. coli TG1 (lac, proA,B, supE, thi, hsdD5 / FtraD36, proA⁺B⁺, lacI^{q}, lacZ, M15), ou E. coli JM101 [Messing et al., Nucl. Acids Res. 9 (1981) 309].

Les souches de levures utilisées appartiennent aux levures bourgeonnantes et plus particulièrement aux levures du genre Kluyveromyces. Les souche K. lactis MW98.8C (a, uraA, arg, lys, K⁺, pKD1°), K. lactis CBS 293.91, K. lactis CBS 294.91 (uraA), et K. lactis CBS 2359/152 [a, metA, (k1, k2) ; Wésolowski et al., Yeast 4 (1988) 71] ont été particulièrement utilisées ; un échantillon de la souche MW98-8C a été déposé le 16 Septembre 1988 au Centraalbureau voor Schimmelkulturen (CBS) à Baarn (Pays Bas) où il a été enregistré sous le numéro CBS 579.88.

La préparation d'ADN génomique de levure est essentiellement dérivée de la technique de Hoffman et Winston [Gene 57 (1987) 267] et est décrite en détail dans le texte.

Les souches de levures transformées par les plasmides d'expression codant pour les protéines de la présente invention sont cultivées en erlenmeyers ou en fermenteurs pilotes de 21 (SETRIC, France) à 28°C en milieu riche (YPD : 1 % yeast extract, 2 % Bactopeptone, 2 % glucose ; ou YPL : 1 % yeast extract, 2 % Bactopeptone, 2 % lactose) sous agitation constante.

### EXEMPLES

### EXEMPLE 1 : CLONAGE DU GENE DE LA PROTEASE B DE K. LACTIS.

### E.1.1. Réalisation d'une sonde par amplification enzymatique

### in vitro d'une séquence d'ADN.

Une amplification enzymatique par PCR est réalisée à partir du plasmide pFP8 [Moehle et al., Genetics 115 (1987) 255 ; Fig. 1], vecteur navette E. coli/S. cerevisiae dérivé de YEp13 et portant le gène PRB1 de S. cerevisiae, et les oligodéoxynucléotides 5'-TGACACTCAAAATAGCG-3' (le codon correspondant au résidu Asp³ est souligné) et 5'-AATATCTCTCACTTGAT-3' (le codon du brin complémentaire correspondant au résidu Ile³⁴⁸ est souligné). La température d'hybridation des oligodéoxynucléotides est de 45°C et le volume réactionel de 100 ml comprend : 10 ng du plasmide pFP8, les oligodéoxynucléotides amorces, 10 ml de tampon 10X PCR [Tris-HCl pH=8,5 (100 mM) ; MgC12 (20 mM) ; KCl (100 mM) ; gélatine (0,01 %)], 10 ml dNTP (dATP + dCTP + dGTP + dTTP, chacun à une concentration de 10 mM)], et 2,5 unités de Taq polymérase. L'addition d'une goutte d'huile de parafine permet d'éviter l'évaporation au cours des élévations de température pendant les cycles d'amplification. Un fragment d'ADN de 1039 paires de bases est obtenu, dont l'identité est vérifiée par analyse des positions de certains sites de restriction et correspondant à la quasi-totalité de la séquence en acides aminés de la forme mature de la protéase B (Asp³ à Ile³⁴⁸). Ce fragment est ensuite purifié par électroélution après migration sur gel d'agarose à 0,8 % et est utilisé pour préparer la sonde radioactive selon la méthode du "Random Priming".

### E.1.2. Préparation d'ADN génomique de levure.

Les levures (souche K. lactis MW98-8C) en phase stationnaire de croissance sont centrifugées. Après lavage du culot dans de l'eau stérile, celui-ci est repris dans une solution contenant : Triton X100 2 % (v/v) ; SDS 1 % (w/v) ; NaCl 100 mM ; Tris-HCl (pH=8) 10 mM ; EDTA 1 mM. Les levures sont alors broyées en présence de phénol-chloroforme par l'action mécanique de billes de verre ajoutées au mélange qui est agité au Vortex pendant 2 minutes. La phase aqueuse est ensuite récupérée après centrifugation et précipitée par addition de 2,5 volumes d'éthanol. L'ADN est repris dans du TE pour subir une purification sur un gradient de césium. A partir de 1 litre de culture on obtient environ 1 mg d'ADN génomique de haut poids moléculaire (>> 20kb).

### E.1.3. Recherche du gène par hybridation en conditions de faible stringence.

La préparation d'ADN génomique est soumise à une digestion totale par des enzymes de restriction dont les sites sont présents dans le multisite de clonage du vecteur pIC-20H. Un résultat préliminaire montre que seules des conditions peu stringentes (hybridation pendant 15 heures à 40°C sans formamide en 5X SSC/5X Denhart, puis 3 lavages en 5X SSC/SDS 1 % à 40°C pendant 15 minutes, puis 1 lavage en 0,25X SSC/SDS 1 % pendant 10 minutes) permettent de visualiser un fragment EcoRI de 1,8 kb hybridant avec la sonde PRB1 de S. cerevisiae. Un second Southern blot est réalisé, dans lequel chaque puits comporte 12 mg d'ADN génomique clivé pendant 15 heures par 20 unités de EcoRI et 20 unités d'une seconde enzyme de restriction (Fig. 2). Dans les conditions d'hybridation et de lavage précédemment définies, un fragment génomique d'environ 700 pb et provenant de la double digestion EcoRI+BglII hybride avec la sonde PRB1 de S. cerevisiae (Fig. 2, puits n° 3). De même, un fragment de restriction BglII de taille supérieure (environ 1,3 kb) hybride avec cette sonde (Fig. 2, puits n° 2). Le fragment d'environ 700 pb détecté après digestion de l'ADN génomique par EcoRI + BglII est donc un fragment de restriction BglII-EcoRI. Les autres restrictions paraissent moins intéressantes car elles génèrent soit des fragments de taille inférieure à celui obtenu par la double digestion EcoRI + BglII. soit un fragment de taille identique (1,8 kb) à celui généré après digestion par EcoRI uniquement (Fig. 2, puits n° 1).

Le clonage de ce fragment asymétrique BglII-EcoRI de 700 pb permet d'obtenir une fraction du gène PRB1 de K. lactis qui pourra servir ensuite de sonde homologue pour cloner la ou les partie(s) manquante(s) du gène. Pour cloner ce fragment, 100 mg d'ADN génomique sont traités pendant 15 heures par 100 unités des endonucléases EcoRI et BglII. puis mis à migrer sur gel d'agarose préparatif (1 %). La fraction du gel comprenant les fragments dont la taille est comprise entre 500 et 1000 pb est ensuite découpée en trois sous-fractions (sous-fraction "f" : 500/700 pb ; sous-fraction "e" : 700/800 pb ; sous-fraction "d" : 800/1000 pb ; Fig. 3). Un Southern blot réalisé après migration d'un aliquot de ces sous-fractions et hybridation avec la sonde PRB1 de S. cerevisiae montre un signal d'hybridation de la taille attendue (700 pb) et particulièrement intense avec la sous-fraction "e" (700/800 pb). Une banque génomique restreinte aux fragments BglII-EcoRI de cette sous-fraction (mini-banque génomique) est donc construite par clonage des fragments de restriction BglII-EcoRI dans les sites correspondant du vecteur pIC-20H. La transformation de la ligation dans E. coli donne 90 % de clones blancs sur boîtes LB supplémentées en ampicilline et X-gal. 340 clones (dont environ 30 clones bleus) sont alors repiqués sur le même milieu pour les isoler. Les 340 clones de la banque restreinte sont ensuite repartis en 34 mélanges correspondant chacun à 10 clones différents et leur ADN est digéré par EcoRI+BglII, migrés en gel d'agarose, transferés sur membrane pour être ensuite hybridés avec la sonde PRB1 de S. cerevisiae en conditions peu stringentes d'hybridation et de lavage. La Figure 3 montre ce Southern blot où deux mélanges (n° 18 et n° 31) présentent un signal d'hybridation de la taille attendue (environ 700 pb). La même opération est faite pour analyser séparément les 10 clones des mélanges n° 18 et n° 31 : le clone n° 3 est le seul clone du mélange n° 18 à présenter un signal d'hybridation à la taille attendue (clone 18-3) ; de façon analogue, seul le clone I du mélange 31 (clone 31-I) donne un signal d'hybridation à la taille attendue. Un dernier Southern blot est réalisé à partir de l'ADN des clones 18-3 et 31-I (signal positif) et d'un clone négatif (clone K du mélange n° 31). Dans les conditions d'hybridation et de lavage définies précédemment, seuls les clones 18-3 et 31-I confirment la présence d'un signal positif après digestion double EcoRI+BglII, et dont la taille semble strictement équivalente (Fig. 4).

### E.1.4. Identification du gène.

La séquence nucléotidique du fragment BglII-EcoRI du clone 18-3 est réalisée pour démontrer que ce fragment correspond bien à une fraction du gène PRB1 de K. lactis.

Une carte de restriction sommaire du plasmide pYG1224 du clone 18-3 est d'abord réalisée et révèle la présence d'un site SalI apparemment unique au centre du fragment BglII-EcoRI. Les fragments BglII-SalI (environ 350 pb) et SalI-EcoRI (environ 300 pb) du plasmide pYG1224 sont alors clonés dans le vecteur pUC19, ce qui génère les plasmides pYG1226 et pYG1227 respectivement (Fig. 6A). Les inserts de ces plasmides sont alors séquencés en entier en utilisant les "amorces universelles". Comme indiqué à la Figure 5, le fragment BglII-EcoRI du plasmide pYG1224 comporte une phase ouverte de lecture (225 résidus) qui présente des homologies de séquences avec un fragment du gène PRB1 de S. cerevisiae (Arg¹⁰⁵ à Leu³²⁸). La présence d'une telle homologie, de même que la stricte conservation des acides aminés invariablement retrouvés dans les sérine-protéases de la famille des subtilisines démontrent que le fragment d'ADN génomique porté par le plasmide pYG1224 correspond bien à un fragment du gène PRB1 de K. lactis.

### E.1.5. Clonage de la partie 3' du gène.

Le fragment BglII-EcoRI du plasmide pYG1224 comporte un site de restriction KpnI unique localisé en aval du site BglII (Fig. 6A). Le sous-fragment de restriction KpnI-EcoRI d'environ 665 nucléotides est donc généré à partir de ce fragment, isolé par électroélution après migration en gel d'agarose à 1 % et marqué radioactivement par la méthode du "random priming". Cette sonde radioactive est alors utilisée pour déterminer la taille des fragments de restriction de l'ADN génomique de K. lactis qui l'incluent. Un fragment KpnI-BglII d'environ 1,2 kb est ainsi détecté après hybridation et lavage en conditions stringentes (hybridation pendant 15 heures à 40°C en 5X SSC/5X Denhart/50 % formamide, puis 3 lavages en 5X SSC/SDS 1 % à 40°C pendant 15 minutes, puis 1 lavage en 0,2X SSC/SDS 1 % pendant 10 minutes). Une banque restreinte d'ADN génomique de K. lactis (fragments de restriction KpnI-BglII de taille comprise entre 1 et 1.5 kb) est alors construite selon l'exemple E.1.3. et le fragment de restriction hybridant avec la sonde est cloné entre les sites KpnI et BamHI du vecteur pIC-20H, ce qui génère le plasmide pYG1231 (Fig. 6B). L'insert génomique de ce plasmide est ensuite séquence en utilisant l'oligodéoxynucléotide Sq2101 (5'-GACCTATGGGGTAAGG-ATTAC-3') comme amorce. Cet oligodéoxynucléotide correspond à une séquence nucléotidique présente sur le fragment BglII-EcoRI du plasmide pYG1224 et localisée à environ 30 nucléotides du site EcoRI. Il permet donc de déterminer la séquence nucléotidique située en 3' de ce site de restriction, et notamment la séquence localisée entre le site EcoRI et le codon spécifiant la terminaison de la traduction de l'ARN messager correspondant au gène PRB1 de K. lactis.

### E.1.6. Clonage de la partie 5' du gène.

La séquence nucléotidique réalisée en E.1.5. démontre l'existence d'un site de restriction HindIII localisé entre le site EcoRI et le codon de fin de traduction. L'utilisation du fragment de restriction KpnI-EcoRI correspondant à la partie C-terminale du gène PRB1 de K. lactis comme sonde radioactive sur de l'ADN génomique de K. lactis digéré par HindIII et une seconde enzyme permet d'identifier par Southern blot un fragment HindIII-EcoRV de 1,7 kb environ qui hybride avec cette sonde. Ce fragment de restriction est d'abord cloné entre les sites EcoRV et HindIII du vecteur pIC-20R ce qui génère le plasmide pYG1237. Une carte de restriction de l'insert d'ADN génomique contenu dans le plasmide pYG1237 est réalisé (Fig. 6C), et les plasmides suivants sont générés : pYG1238 (plasmide pYG1237 délété de son fragment PstI), pYG1239 (fragment PstI de pYG1237 dans le vecteur pUC19), pYG1240 (plasmide pYG1237 délété de son fragment KpnI), pYG1241 (plasmide pYG1237 délété de son fragment ClaI) et pYG1242 (plasmide pYG1237 délété de son fragment SalI; Fig. 6C). Les inserts génomiques de ces différents plasmides sont alors séquencés à l'aide des amorces universelles et de l'oligodéoxynucléotide Sq2148 (5'-GCTTCGGCAACATATTCG-3') qui permet de séquencer la région située immédiatement en 5' du site BglII. Cette stratégie permet d'obtenir des séquences chevauchantes démontrant l'unicité des sites de restriction BglII, ClaI et PstI et permettant d'identifier l'ATG probable d'initiation de la traduction du gène PRB1 de K. lactis.

### E.1.7. Séquence nucléotidique du gène PRB1 de K. lactis.

La compilation des séquences déterminées en E.1.4., E.1.5. et E.1.6. recouvre l'intégralité de la phase codante du gène PRB1 de K. lactis (Fig. 6D). Cette séquence est donnée SEQ ID n° 1 et code pour une protéine de 561 résidus correspondant à la protéase B de K. lactis.

### EXEMPLE 2 : CLONAGE DU GENE DE LA CARBOXYPEPTIDASE Y DE K. LACTIS.

La stratégie générale décrite dans l'exemple 1 est reprise pour le clonage du gène de la carboxypeptidase Y de K. lactis CBS 2359/152.

### E.2.1. Préparation de la sonde.

Une préparation d'ADN génomique de la souche S. cerevisiae S288C [Mortimer et Johnston, Genetics 113 (1986) 35] est d'abord effectuée selon l'exemple E.1.2. Une amplification par PCR de cette préparation d'ADN génomique est ensuite réalisée avec les oligonucléotides 5'-CTTCTTGGAGTTGTTCTTCG-3' et 5'-TGGCAAGACATCCGTCCACGCCTTATT-ACC-3', spécifiques du gène PRC1. Un fragment amplifié de la taille attendue (699 pb) est ainsi obtenu qui correspond aux positions 696-1395 (le codon d'initiation ATG étant numéroté +1) de la phase ouverte de lecture du gène PRC1 de S. cerevisiae [Valls et al., Cell 48 (1987) 887]. Ce fragment est ensuite purifié par électroélution et radiomarqué selon la technique du "Random Priming".

### E.2.2. Clonage du gène PRC1 de K. lactis.

Le gène PRC1 de K. lactis est obtenu par criblage de la banque génomique de K. lactis construite par Wésolowski-Louvel [Yeast 4 (1988) 71] à partir de la souche 2359/152 dans le vecteur de clonage KEp6 [Chen et al., J. Basic. Microbiol. 28 (1988) 211]. La souche E.coli JM101 est transformée par l'ADN de la banque et les transformants sont étalés sur milieu LB supplémenté avec de l'ampicilline (50 mg/l). 15000 clones sont ensuite transférés sur filtres de nitrocellulose et les filtres sont hybridés avec la sonde décrite dans l'exemple E.2.1. Les conditions d'hybridation et de lavage sont celles de l'exemple E.1.3. 12 clones positifs sont ainsi isolés et l'un d'entre-eux, désigné pC34, est ensuite retenu pour la suite de l'étude. Dans un premier temps, l'hybridation du plasmide pC34 avec la sonde correspondant au gène PRC1 de S. cerevisiae est confirmée en Southern blot. Une carte de restriction de l'insert génomique (environ 6,9 kb) du plasmide pC34 est donnée à la Figure 8. La séquence du fragment EcoRI-SalI de 2,5 kb comprenant le gène PRC1 de K. lactis est ensuite déterminée sur les 2 brins. Cette séquence est présentée SEQ ID n° 2.

### EXEMPLE 3 : CLONAGE DU GENE DE LA PROTEASE A DE K. LACTIS.

La stratégie générale décrite dans les exemples précédents est reprise pour le clonage du gène de la protéase A de K. lactis CBS 2359/152.

### E.3.1. Préparation de la sonde.

Un fragment interne de 449 pb du gène PRA1 (ou gène PEP4) de S. cerevisiae est d'abord amplifié par la technique PCR à partir du plasmide CBZ1B1 [Woolford et al., Mol. Cell. Biol. 6 (1986) 2500] fourni par le Dr E. Jones (Carnegie-Mellon University, Pittsburgh, Pennsylvania, USA) et des oligodéoxynucléotides 5'-CTGTTGATAAGGTGGTCC-3' et 5'-CAAGCGT-GTAATCGTATGGC-3'. Le fragment amplifié obtenu correspond aux positions 617-1066 de la phase ouverte de lecture du gène PRA1 de S. cerevisiae, le codon d'initiation ATG étant numéroté +1. Ce fragment est ensuite purifié par électroélution et radiomarqué selon la technique du "Random Priming".

### E.3.2. Clonage du gène PRA1 de K. lactis

Le gène PRA1 est obtenu par criblage de la banque génomique de K. lactis construite par Wésolowski-Louvel [Yeast 4 (1988) 71] à partir de la souche 2359/152 dans le vecteur de clonage KEp6 [Chen et al., J. Basic Microbiol. 28 (1988) 211]. Après transformation de la banque dans E. coli JM101 et sélection en présence d'ampicilline (50 mg/l), 15000 clones sont ensuite transférés sur filtres de nitrocellulose et les filtres sont hybridés avec la sonde décrite dans l'exemple E.3.1. Les conditions d'hybridation et de lavage sont celles de l'exemple E.1.3. 1 seul clone positif est ainsi isolé et désigné pA25/1. Dans un premier temps, l'hybridation du plasmide pA25/1 avec la sonde correspondant au gène PRA1 de S. cerevisiae est confirmée en Southern blot. Une carte de restriction de l'insert génomique (environ 7,5 kb) de ce plasmide est présentée à la Figure 10. La séquence du fragment ClaI-EcoRI de 1,6 kb comprenant le gène PRA1 de K. lactis est ensuite déterminée sur les 2 brins. Cette séquence est présentée SEQ ID n° 3.

### EXEMPLE 4 : TRANSFORMATION DES LEVURES.

La transformation des levures appartenant au genre Kluyveromyces, et en particulier les souches K. lactis MW98-8C, CBS 293.91 et CBS 294.91 (uraA) s'effectue par exemple par la technique de traitement des cellules entières par de l'acétate de lithium [Ito H. et al., J. Bacteriol. 153 (1983) 163-168], adaptée comme suit. La croissance des cellules se fait à 28°C dans 50 ml de milieu YPD. avec agitation et jusqu'à une densité optique à 600 nm (DO₆₀₀) comprise entre 0,6 et 0,8; les cellules sont alors récoltées par centrifugation à faible vitesse, lavées dans une solution stérile de TE (10 mM Tris HCl pH 7,4 ; 1 mM EDTA), resuspendues dans 3-4 ml d'acétate lithium (0,1 M dans du TE) pour obtenir une densité cellulaire d'environ 2 x 10⁸ cellules/ml, puis incubées à 30°C pendant 1 heure sous agitation modérée. Des aliquotes de 0,1 ml de la suspension résultante de cellules compétentes sont incubés à 30°C pendant 1 heure en présence d'ADN et à une concentration finale de 35 % de polyéthylène glycol (PEG₄₀₀₀, Sigma). Après un choc thermique de 5 minutes à 42°C, les cellules sont lavées 2 fois, puis resuspendues dans 0,2 ml d'eau stérile. Dans le cas ou le marqueur de sélection est le gène URA3 de S. cerevisiae, les cellules sont directement étalées sur YNB (Yeast Nitrogen Base ; Difco)/Glucose (20 g/l)/agar. Dans le cas ou le marqueur de sélection est le gène aph du transposon Tn903, les cellules sont d'abord incubées pendant 16 heures à 28°C dans 2 ml de milieu YPD pour permettre l'expression phénotypique du géne de résistance au G418 exprimé sous contrôle du promoteur Pₖ₁ (cf. EP 361 991) ; 200 µl de la suspension cellulaire sont ensuite étalés sur boîtes YPD sélectives (G418, 200 µg/ml). Les boîtes sont mises à incuber à 28°C et les disruptants ou les transformants apparaissent après 2 à 3 jours de croissance cellulaire.

### EXEMPLE 5 : DISRUPTION DE GENES DE PROTEASES CHEZ K. LACTIS.

### E.5.1. Souches de K. lactis disruptées pour le gène PRB1.

Le plasmide pYG1229 est construit par clonage du fragment HindIII-EcoRI (incluant le fragment BglII-EcoRI d'environ 700 bp et correspondant à la partie C-terminale du gène PRB1 de K, lactis) du plasmide pYG1224 entre les sites correspondant du plasmide pUC9. Le plasmide pYG1228 est construit par clonage du fragment HindIII de 1,1 kb et correspondant au gène URA3 de S. cerevisiae provenant du plasmide pCG3 [Gerbaud et al., Curr. Genetics 3 (1981) 173] dans le site HindIII du plasmide pIC-20R. Le plasmide pYG1228 permet donc de disposer d'un fragment de restriction SalI-XhoI d'environ 1,1 kb et contenant l'intégralité du fragment HindIII contenant le gène URA3 de S. cerevisiae. Ce fragment de restriction est ensuite cloné dans le site SalI du plasmide pYG1229 ce qui génère le plasmide pYG1232 (2 orientations possibles). La digestion de ce plasmide par les enzymes BglII et EcoRI permet de générer un fragment de restriction d'environ 1,8 kb correspondant au gène URA3 de S. cerevisiae bordé par des séquences génomiques de K. lactis provenant du gène PRB1 (Fig. 11A). La transformation de mutants uraA de K. lactis par le fragment BglII-EcoRI du plasmide pYG1232 génère des clones transformés (complémentés par le gène URA3 de S. cerevisiae) correspondant à l'intégration de ce fragment dans le chromosome. Le panneau B de la Figure 11 montre l'intégration de ce fragment dans l'ADN génomique de la souche K. lactis CBS 294.91 (uraA) après recombinaison non-homologue (puits 1) ou après recombinaison homologue dans le gène PRB1 (puits 3, ce disruptant est noté Y750). La disruption de l'allèle sauvage du gène PRB1 ne modifie pas les caractéristiques de croissance de la souche.

### E.5.2. Souches de K. lactis disruptées pour le gène PRC1.

Le fragment SalI-SphI de 4,4 kb provenant du plasmide pC34 est d'abord sous-cloné dans les sites correspondants du vecteur pIC-20R, ce qui génère le plasmide pYG154 (Fig. 12, panneau b). Ce plasmide est ensuite digéré par l'enzyme SphI, puis traité avec l'ADN polymérase I du phage T4 en présence de phosphatase intestinale de veau (CIP). Le plasmide obtenu est ensuite ligaturé avec le fragment EcoRI de 1,6 kb portant le gène URA3 de S. cerevisiae provenant du plasmide pKan707 (EP 361 991), préalablement traité avec le fragment Klenow de l'ADN polymérase I d'E. coli. Le plasmide obtenu est désigné pYG155 (Fig. 12, panneau c). Le fragment SalI-BamHI de 4,5 kb du plasmide pYG155 est ensuite purifié par électroélution et utilisé pour transformer la souche K. lactis CBS 294.91 (uraA). Les transformants sont sélectionnés pour le phénotype Ura⁺ et quelques clones sont ensuite analysés en Southern blot pour vérifier le lieu d'intégration du marqueur URA3. Le clone Y797 est ainsi identifié dans lequel le gène PRC1 chromosomique a été remplacé, par recombinaison homologue, par l'allèle disrupté construit in vitro. La disruption de l'allèle sauvage du gène PRC1 ne modifie pas les caractéristiques de croissance de la souche.

### EXEMPLE 6 : PLASMIDES D'EXPRESSION.

### E.6.1. Plasmide pYG1212.

Les gènes des protéines d'intérêt que l'on souhaite sécréter et/ou exprimer sont d'abord insérés, "dans l'orientation productive" (définie comme l'orientation qui place la région N-terminale de la protéine de façon proximale par rapport au promoteur de transcription), sous contrôle de promoteurs fonctionnels, régulables ou constitutifs, tels que, par exemple, ceux présents dans les plasmides pYG105 (promoteur LAC4 de K, lactis), pYG106 (promoteur PGK de S.cerevisiae), pYG536 (promoteur PHO5 de S. cerevisiae), ou des promoteur hybrides tels que ceux décrits dans la demande de brevet EP 361 991. Les plasmides pYG105 et pYG106 sont particulièrement utiles car ils permettent l'expression de gènes inclus dans des fragments de restriction HindIII à partir de promoteurs fonctionnels chez K. lactis, régulables (pYG105) ou constitutifs (pYG106).

Le plasmide pYG105 correspond au plasmide pKan707 décrit dans la demande de brevet EP 361 991 dans lequel le site de restriction HindIII unique et localisé dans le gène de résistance à la généticine (G418) a été détruit par mutagénèse dirigée tout en conservant une protéine inchangée (oligodéoxynucleotide 5'-GAAATGCATAAGCTCTTGCCATTCTCACCG-3'). Le fragment SalI-SacI codant pour le gène URA3 du plasmide ainsi muté a été ensuite remplacé par un fragment de restriction SalI-SacI comportant une cassette d'expression constituée du promoteur LAC4 de K. lactis [sous la forme d'un fragment SalI-HindIII provenant du plasmide pYG107 ; Fleer et al., Bio/Technology 9 (1991) 968]) et du terminateur du gène PGK de S. cerevisiae [sous la forme d'un fragment HindIII-SacI ; Fleer et al., Bio/Technology 2 (1991) 968]. Le plasmide pYG105 est mitotiquement très stable chez les levures Kluyveromyces et une carte de restriction en est donnée à la Figure 13. Les plasmides pYG105 et pYG106 ne diffèrent entre eux que par la nature du promoteur de transcription encodé par le fragment SalI-HindIII.

La protéine codée par le plasmide pYG1212 correspond approximativement aux deux premiers domaines de la sérum albumine humaine (SAH). Ce variant moléculaire, obtenu par digestion de l'extrémité C-terminale de la SAH en utilisant l'exonucléase Ba131 à partir du site MstII unique localisé à 3 acides aminés de l'extrémité C-terminale de la SAH, est dérivé du plasmide YP40 décrit dans la demande de brevet EP 413 622. Brièvement, le fragment de restriction HindIII-MstII du plasmide YP40, correspondant aux résidus 1 à 403 de la SAH (l'ATG d'initiation de la traduction est noté +1), est ligaturé avec le fragment MstII-HindIII du plasmide pYG221 [Yeh et al., Proc. Natl. Acad. Sci. USA 89 (1992) 1904], ce qui génère un fragment HindIII incluant les 403 résidus N-terminaux de la SAH suivi des trois derniers résidus de la SAH (résidus Leu-Gly-Leu) et d'un codon de fin de traduction [variant tronqué noté SAH₍₁₋₄₀₃₎]. Ce fragment-HindIII est ensuite cloné dans l'orientation productive dans le plasmide pYG105, ce qui génère le plasmide pYG1212 (Fig.13).

### EXEMPLE 7 : POTENTIEL DE SECRETION DES DISRUPTANTS.

### E.7.1. SAH₍₁₋₄₀₃₎ dans un disruptant pour la protéase B.

Dans un premier temps, les levures K. lactis CBS 293.91 et Y750 sont transformées par le plasmide pYG1212. Après sélection sur milieu riche supplémenté en G418 les clones recombinants sont testés pour leur capacité à sécréter la protéine SAH₍₁₋₄₀₃₎. Quelques clones sont mis à incuber en milieu YPD ou YPL à 28°C. Les surnageants de culture sont récupérés par centrifugation quand les cellules atteignent la phase stationnaire de croissance, concentrés 10 fois par précipitation pendant 30 minutes à -20°C dans une concentration finale de 60 % d'éthanol, puis testés après électrophorèse en gel SDS-PAGE à 8.5 % et coloration du gel par du bleu de coomassie. Les résultats présentés à la Figure 14 démontrent que le disruptant Y750 (prb1°) sécrète des quantités de la protéine qui sont bien supérieures aux quantités sécrétées par son homologue non disrupté. Ceci est valable après 2, 4 ou 7 jours de croissance, indépendamment de la source de carbone utilisée (glucose ou lactose).

## Revendications

1. Levure du genre Kluyveromyces présentant une ou plusieurs modifications génétiques d'au moins un gène codant pour une protéase choisie parmi la protéase A représentée par la séquence polypeptidique SEQ ID N°3, la protéase B représentée par la sequence polypeptique SEQ ID N°1 et la carboxypeptidase Y, représentée par la séquence SEQ ID N°2 lesdites modifications génétiques diminuant l'activité protéolytique desdites levures.

2. Levure selon la revendication 1 **caractérisée en ce que** la ou les modifications génétiques rendent ledit gène partiellement ou totalement incapable de coder pour la protéine naturelle.

3. Levure selon la revendication 1 **caractérisée en ce que** le ou les gènes ainsi modifiés génétiquement codent pour une protéine non fonctionnelle ou pour un mutant ayant un spectre d'activité protéolytique modifié.

4. Levure selon les revendications 1 à 3 **caractérisée en ce que** la ou les modifications génétiques sont stables ségrégationellement.

5. Levure selon les revendications 1 à 4 **caractérisée en ce que** la ou les modifications génétiques sont non-réversibles.

6. Levure selon les revendications 1 à 5 **caractérisée en ce que** la ou les modifications ne laissent aucune activité résiduelle au gène considéré.

7. Levure selon les revendications 1 à 6 **caractérisée en ce que** la ou les modifications portent sur la partie codant pour la protéine ayant une activité de protéase et/ou sur les régions responsables de l'expression et/ou de la régulation transcriptionnelle desdits gènes.

8. Levure selon l'une des revendications 1 à 7 **caractérisée en ce que** la ou les modifications génétiques sont des mutations ponctuelles ou multiples et/ou des additions et/ou des délétions et/ou des disruptions.

9. Levure selon l'une des revendications 1 à 8, **caractérisée en ce qu'**il s'agit d'un gène de Kluyveromyces choisi parmi les gènes PRB1 (SEQ ID N°1), PRC1 (SEQ ID N°2) et PRA1 (SEQ ID N°3).

10. Levure selon l'une quelconque des revendications 1 à 9 **caractérisée en ce qu'**elle est choisie parmi les levures appartenant aux espèces K. lactis, K. fragilis, K. drosophilarum, et K. waltii.

11. Levure du genre Kluyveromyces **caractérisée en ce qu'**elle présente une ou plusieurs modifications génétiques d'au moins un gène codant pour une protéase choisie parmi la protéase A représentée par la séquence polypeptidique SEQ ID N°3, la protéase B représentée par la séquence polypeptique SEQ ID N°1 et la carboxypeptidase Y, représentée par la séquence SEQ ID N°2, lesdites modifications génétiques diminuant l'activité protéolytique desdites levures, et **en ce qu'**elle comprend en plus une séquence d'ADN exogène codant au moins pour une protéine d'intérêt.

12. Levure selon la revendication 11 **caractérisée en ce que** la séquence d'ADN exogène comprend une région de démarrage de la transcription et de la traduction jointe à l'extrémité 5' de la séquence codant pour la protéine d'intérêt.

13. Levure selon l'une des revendications 11 ou 12 **caractérisée en ce que** la séquence codant pour la protéine d'intérêt inclus une séquence d'exportation dirigeant la protéine dans la voie sécrétoire.

14. Levure selon l'une des revendications 11 à 13 **caractérisée en ce que** la séquence d'ADN exogène fait partie d'un vecteur à réplication autonome ou est intégrée dans le chromosome.

15. Levure selon l'une des revendications 11 à 14 **caractérisée en ce que** la ou les protéines d'intérêt sont à usage pharmaceutique ou agroalimentaire.

16. Levure selon la revendication 15 **caractérisée en ce que** la ou les protéines sont des protéines artificielles et par exemple hybrides.

17. Utilisation d'une levure selon l'une des revendications 1 à 16 pour la production de protéines recombinantes.

18. Procédé de production de protéines recombinantes **caractérisé en ce que** l'on cultive une levure selon l'une des revendications 11 à 16 dans des conditions d'expression de la ou des protéines codées par la séquence d'ADN exogène et que l'on récupère la ou les protéines d'intérêt.

19. Procédé selon la revendication 18 pour la production de protéines d'intérêt pharmaceutique ou agroalimentaire.

20. Procédé selon la revendication 19 pour la production de protéines artificielles et notamment hybrides.

21. Procédé de préparation d'une levure Kluyveromyces selon la revendication 1 **caractérisé en ce que** l'on remplace tout ou partie des gènes chromosomiques PRB1 (SEQ ID N°1), PRC1 (SEQ ID N°2) ou PRA1 (SEQ ID N°3) par une version modifiée in vitro.

## Claims

1. Yeast of the genus Kluyveromyces having one or more genetic modifications of at least one gene encoding a protease chosen from protease A represented by the polypeptide sequence SEQ ID No. 3, protease B represented by the polypeptide sequence SEQ ID No. 1 and carboxypeptidase Y represented by the sequence SEQ ID No. 2, the said genetic modifications reducing the proteolytic activity of the said yeasts.

2. Yeast according to Claim 1, **characterized in that** the genetic modification(s) render the said gene partially or totally incapable of encoding the natural protein.

3. Yeast according to Claim 1, **characterized in that** the gene(s) thus genetically modified encode a non-functional protein or a mutant having a modified proteolytic activity spectrum.

4. Yeast according to Claims 1 to 3, **characterized in that** the genetic modification(s) are segregationally stable.

5. Yeast according to Claims 1 to 4, **characterized in that** the genetic modification(s) are non-reversible.

6. Yeast according to Claims 1 to 5, **characterized in that** the modification(s) do not leave any residual activity for the gene considered.

7. Yeast according to Claims 1 to 6, **characterized in that** the modification(s) affect the part encoding the protein having a protease activity and/or the regions responsible for the expression and/or the transcriptional regulation of the said genes.

8. Yeast according to one of Claims 1 to 7, **characterized in that** the genetic modification(s) are point or multiple mutations and/or additions and/or deletions and/or disruptions.

9. Yeast according to one of Claims 1 to 8, **characterized in that** it involves a Kluyveromyces gene chosen from the genes PRBI (SEQ ID No. 1), PRC1 (SEQ ID No. 2) and PRA1 (SEQ ID No. 3).

10. Yeast according to any one of Claims 1 to 9, **characterized in that** it is chosen from yeasts belonging to the species K. lactis, K. fragilis, K. drosophilarum, and K. waltii.

11. Yeast of the genus Kluyveromyces, **characterized in that** it has one or more genetic modifications of at least one gene encoding a protease chosen from protease A represented by the polypeptide sequence SEQ ID No. 3, protease B represented by the polypeptide sequence SEQ ID No. 1 and carboxypeptidase Y represented by the sequence SEQ ID No. 2, the said genetic modifications reducing the proteolytic activity of the said yeasts, and **in that** it comprises in addition an exogenous DNA sequence encoding at least one protein of interest.

12. Yeast according to Claim 11, **characterized in that** the exogenous DNA sequence comprises a region for initiation of transcription and translation joined to the 5' end of the sequence encoding the protein of interest.

13. Yeast according to either of Claims 11 and 12, **characterized in that** the sequence encoding the protein of interest includes an exporting sequence directing the protein in the secretory pathway.

14. Yeast according to one of Claims 11 to 13, **characterized in that** the exogenous DNA sequence is part of an autonomously replicating vector or is integrated into the chromosome.

15. Yeast according to one of Claims 11 to 14, **characterized in that** the protein(s) of interest are for pharmaceutical or dietary use.

16. Yeast according to Claim 15, **characterized in that** the protein(s) are artificial proteins and for example hybrid proteins.

17. Use of a yeast according to one of Claims 1 to 16 for the production of recombinant proteins.

18. Process for producing recombinant proteins, **characterized in that** a yeast according to one of Claims 11 to 16 is cultured under conditions for expressing the protein(s) encoded by the exogenous DNA sequence and **in that** the protein(s) of interest is (are) recovered.

19. Process according to Claim 18, for the production of proteins of pharmaceutical or dietary interest.

20. Process according to Claim 19, for the production of artificial proteins and in particular hybrid proteins.

21. Process for preparing a Kluyveromyces yeast according to Claim 1, **characterized in that** all or part of the chromosomal genes PRBI (SEQ ID No. 1), PRC1 (SEQ ID No. 2) or PRAl (SEQ ID No. 3) are replaced by a modified version in vitro.

## Patentansprüche

1. Hefe der Gattung Kluyveromyces, die eine oder mehrere genetische Modifikationen in mindestens einem Gen aufweist, das für eine Protease codiert, die ausgewählt ist aus Protease A, dargestellt durch die Polypeptidsequenz SEQ ID NR. 3, Protease B, dargestellt durch die Polypeptidsequenz SEQ ID NR. 1, und Carboxypeptidase Y, dargestellt durch die Sequenz SEQ ID NR. 2, wobei diese genetischen Modifikationen die proteolytische Aktivität dieser Hefen verringern.

2. Hefe nach Anspruch 1, **dadurch gekennzeichnet, dass** die genetische(n) Modifikation oder Modifikationen das Gen teilweise oder vollständig unfähig machen, für das natürliche Protein zu codieren.

3. Hefe nach Anspruch 1, **dadurch gekennzeichnet, dass** das oder die derart genetisch modifizierte(n) Gen oder Gene für ein nicht-funktionelles Protein oder für eine Mutante mit einem modifizierten proteolytischen Aktivitätsspektrum codiert/codieren.

4. Hefe nach den Ansprüchen 1 bis 3, **dadurch gekennzeichnet, dass** die genetische(n) Modifikation oder Modifikationen segregationsstabil sind.

5. Hefe nach den Ansprüchen 1 bis 4, **dadurch gekennzeichnet, dass** die genetische(n) Modifikation oder Modifikationen irreversibel ist/sind.

6. Hefe nach den Ansprüchen 1 bis 5, **dadurch gekennzeichnet, dass** die genetische(n) Modifikation oder Modifikationen keinerlei Restaktivität des betreffenden Gens belässt/belassen.

7. Hefe nach den Ansprüchen 1 bis 6, **dadurch gekennzeichnet, dass** die genetische(n) Modifikation oder Modifikationen sich auf den Teil, der für ein Protein mit einer Proteaseaktivität codiert, und/oder auf Regionen, die für die Expression und/oder Transkriptionsregulation dieser Gene verantwortlich sind; erstreckt/erstrecken.

8. Hefe nach den Ansprüchen 1 bis 7, **dadurch gekennzeichnet, dass** die genetische(n) Modifikation oder Modifikationen Punktmutationen oder Mehrfachmutationen und/oder Additionen und/oder Deletionen und/oder Disruptionen ist/sind.

9. Hefe nach einem der Ansprüche 1 bis 8, **dadurch gekennzeichnet, dass** es sich um ein Gen von Kluyveromyces handelt, das aus den Genen PRB1 (SEQ ID NR. 1), PRC1 (SEQ ID NR. 2) und PRA1 (SEQ ID NR. 3) ausgewählt ist.

10. Hefe nach einem der Ansprüche 1 bis 9, **dadurch gekennzeichnet, dass** sie aus den Hefen der Spezies K. lactis, K. fragilis, K. drosophilarum und K. waltii ausgewählt ist.

11. Hefe der Gattung Kluyveromyces, **dadurch gekennzeichnet, dass** sie eine oder mehrere genetische Modifikationen in mindestens einem Gen aufweist, das für eine Protease codiert, die ausgewählt ist aus Protease A, dargestellt durch die Polypeptidsequenz SEQ ID NR. 3, Protease B, dargestellt durch die Polypeptidsequenz SEQ ID NR. 1, und Carboxypeptidase Y, dargestellt durch die Sequenz SEQ ID NR. 2, wobei diese genetischen Modifikationen die proteolytische Aktivität dieser Hefen verringern, und **dadurch**, dass sie mindestens eine exogene DNA-Sequenz umfasst, die für mindestens ein Protein von Interesse codiert.

12. Hefe nach Anspruch 11, **dadurch gekennzeichnet, dass** die exogene DNA-Sequenz eine Transkriptions- und Translationsstartregion, angefügt an das 5'-Ende der Sequenz, die für das Protein von Interesse codiert, enthält.

13. Hefe nach einem der Ansprüche 11 oder 12, **dadurch gekennzeichnet, dass** die für das Protein von Interesse codierende Sequenz eine Exportsequenz enthält, die das Protein in den sekretorischen Weg dirigiert.

14. Hefe nach einem der Ansprüche 11 bis 13, **dadurch gekennzeichnet, dass** die exogene DNA-Sequenz Teil eines autonom replizierenden Vektors oder in das Chromosom integriert ist.

15. Hefe nach einem der Ansprüche 11 bis 14, **dadurch gekennzeichnet, dass** das oder die Protein(e) von Interesse pharmazeutisch oder in der Landwirtschaft/Nahrungsmittelindustrie verwendet werden.

16. Hefe nach Anspruch 15, **dadurch gekennzeichnet, dass** das oder die Protein(e) künstliche Proteine und beispielsweise Hybride sind.

17. Verwendung einer Hefe nach einem der Ansprüche 1 bis 16 zur Herstellung rekombinanter Proteine.

18. Verfahren zur Herstellung rekombinanter Proteine, **dadurch gekennzeichnet, dass** man eine Hefe nach einem der Ansprüche 11 bis 16 unter Bedingungen der Expression des oder der Protein(s/e), das/die von der exogenen DNA-Sequenz codiert wird/werden, kultiviert und das oder die Protein(e) von Interesse gewinnt.

19. Verfahren nach Anspruch 18 zur Herstellung von Proteinen, die pharmazeutisch oder für die Landwirtschaft/Nahrungsmittelindustrie von Interesse sind.

20. Verfahren nach Anspruch 19 zur Herstellung künstlicher und insbesondere hybrider Proteine.

21. Verfahren zur Herstellung einer Kluyveromyces-Hefe nach Anspruch 1, **dadurch gekennzeichnet, dass** man das gesamte oder einen Teil der chromosomalen Gene PRB1 (SEQ ID NR. 1), PRC1 (SEQ ID NR. 2) oder PRA1 (SEQ ID NR. 3) durch eine in vitro modifizierte Version ersetzt.


### LISTE DE SEQUENCES

(1) INFORMATION GENERALE:
   (i) DEPOSANT:
      (A) NOM: RHONE-POULENC RORER S.A.
      (B) RUE: 20, avenue Raymond ARON
      (C) VILLE: ANTONY
      (E) PAYS: FRANCE
      (F) CODE POSTAL: 92165
   (ii) TITRE DE L' INVENTION: Levures Kluyveromyces modifiées, préparation et utilisations.
   (iii) NOMBRE DE SEQUENCES: 3
   (iv) FORME LISIBLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 1686 paires de bases
      (B) TYPE: acide nucléique
      (C) NOMBRE DE BRINS: double
      (D) CONFIGURATION: linéaire
   (ii) TYPE DE MOLECULE: ADNc
   (vi) ORIGINE:
      (B) SOUCHE: Kluyveromyces lactis
   (ix) CARACTERISTIQUE ADDITIONELLE:
      (A) NOM/CLE: CDS
      (B) EMPLACEMENT: 1..1686
      (D) AUTRES RENSEIGNEMENTS: /product= "Gene de la protease B" /gene= "K1.PRB1"
   (xi) DESCRIPTION DE LA SEQUENCE: SEQ ID NO: 1:
(2) INFORMATION POUR LA SEQ ID NO: 2:
   (i) CARACTERISTIQUES DE LA SEQUENCE:
      (A) LONGUEUR: 2503 paires de bases
      (B) TYPE: acide nucléique
      (C) NOMBRE DE BRINS: double
      (D) CONFIGURATION: linéaire
   (ii) TYPE DE MOLECULE: ADNc
   (vi) ORIGINE:
   (B) SOUCHE: Kluyveromyces lactis
   (ix) CARACTERISTIQUE ADDITIONELLE:
      (A) NOM/CLE: CDS
      (B) EMPLACEMENT: 387..1862
      (D) AUTRES RENSEIGNEMENTS: /product= "Gene de la protease C de K.lactis" /gene= "Kl.PRC1"
   (xi) DESCRIPTION DE LA SEQUENCE: SEQ ID NO: 2:
   GAATTCTGTC AACTGGATAC GGAAGACAAT AGAATGGACA CATAATGGTC TCAATACGAC      60
   AATTCAACGG CTCTTAGAAG GTGAGTTATT CTTGACATTT TCATGGCTCT TCGAGCATGC     120
   TTTCTAAGAT GACGCGGAAG GTGAAAAAGA TTAGAAAACG GCCATTCACG TGAATATCAC     180
   GTGAACTACA AATTCATGAT ATATTACCGC CAATAGTATT GGTGGTTACC CGATCGTATC     240
   GAATGTACTG ACTTCGAAAA TATGAATAGT CCTCTTTAAA ACAAAGGGTT TTCAGTGACC     300
   CTTACTCCAT CATCTCCTTA GTATTTGGTC TACAGACTCG CCATTGCCGT ATATTCAGGG     360
   TAGTAGTCAG TACATCGGTG TCTGCC ATG GTT TCG ATA AAG TTT CTT TTA TCT     413
                                Met Val Ser Ile Lys Phe Leu Leu Ser
                                  1               5
   TTA TAC GGC TGG CTA TCT GTC ACT TTA GCC ATC TCG TTG AAT GCC GTT      461
   Leu Tyr Gly Trp Leu Ser Val Thr Leu Ala Ile Ser Leu Asn Ala Val
    10                  15                  20                  25
   GTT GAT AGT TTA TTC TCG AAC AGT TTC GAC GGG AAT AAC AAC ATC GAG      509
   Val Asp Ser Leu Phe Ser Asn Ser Phe Asp Gly Asn Asn Asn Ile Glu
                    30                  35                  40
   GAT CAT GAA ACT GCA AAT TAT AAC ACT CAG TTT AGT GTC TTC AGC TCA      557
   Asp His Glu Thr Ala Asn Tyr Asn Thr Gln Phe Ser Val Phe Ser Ser
                45                  50                  55
   AAT ATT GAC GAC GCT TAT TCA TTG AGA ATT AAA CCT TTG GAT CCC AAA      605
   Asn Ile Asp Asp Ala Tyr Ser Leu Arg Ile Lys Pro Leu Asp Pro Lys
            60                  65                  70
   TCT CTT GGC GTT GAT ACC GTG AAA CAA TGG TCG GGA TAT TTA GAT TAC      653
   Ser Leu Gly Val Asp Thr Val Lys Gln Trp Ser Gly Tyr Leu Asp Tyr
        75                  80                  85
   CAG GAC TCA AAA CAC TTC TTT TAT TGG TTT TTT GAG TCT AGA AAT GAC      701
   Gln Asp Ser Lys His Phe Phe Tyr Trp Phe Phe Glu Ser Arg Asn Asp
    90                  95                 100                 105
   CCA GAG AAT GAC CCA GTG ATA CTA TGG TTA AAC GGT GGT CCT GGC TGT      749
   Pro Glu Asn Asp Pro Val Ile Leu Trp Leu Asn Gly Gly Pro Gly Cys
                   110                 115                 120
   TCC TCT TTC GTC GGT CTT TTC TTT GAA TTG GGA CCT TCT TCT ATA GGA      797
   Ser Ser Phe Val Gly Leu Phe Phe Glu Leu Gly Pro Ser Ser Ile Gly
               125                 130                 135
   GCT GAT TTG AAA CCC ATT TAT AAC CCC TAC TCT TGG AAT TCC AAC GCT      845
   Ala Asp Leu Lys Pro Ile Tyr Asn Pro Tyr Ser Trp Asn Ser Asn Ala
           140                 145                 150
   TCT GTG ATA TTC CTA GAT CAG CCT GTT GGT GTT GGG TTC TCA TAC GGT      893
   Ser Val Ile Phe Leu Asp Gln Pro Val Gly Val Gly Phe Ser Tyr Gly
       155                 160                 165
   GAC TCT AAA GTG TCT ACT ACA GAT GAC GCT GCC AAA GAC GTT TAC ATA      941
   Asp Ser Lys Val Ser Thr Thr Asp Asp Ala Ala Lys Asp Val Tyr Ile
   170                 175                 180                 185
   TTC TTA GAT TTG TTC TTT GAA AGA TTC CCT CAT TTG AGA AAT AAC GAT      989
   Phe Leu Asp Leu Phe Phe Glu Arg Phe Pro His Leu Arg Asn Asn Asp
                   190                 195                 200
   TTC CAT ATC TCC GGT GAA TCA TAC GCC GGT CAT TAT TTA CCC AAG ATT     1037
   Phe His Ile Ser Gly Glu Ser Tyr Ala Gly His Tyr Leu Pro Lys Ile
               205                 210                 215
   GCT CAT GAG ATT GCT GTA GTG CAT GCT GAG GAT TCC TCC TTC AAT CTA     1085
   Ala His Glu Ile Ala Val Val His Ala Glu Asp Ser Ser Phe Asn Leu
           220                 225                 230
   TCG TCA GTA TTA ATT GGA AAT GGA TTT ACT GAC CCA CTG ACT CAA TAC     1133
   Ser Ser Val Leu Ile Gly Asn Gly Phe Thr Asp Pro Leu Thr Gln Tyr
       235                 240                 245
   CAA TAT TAC GAG CCG ATG GCC TGT GGT GAA GGT GGT TAT CCA GCG GTG     1181
   Gln Tyr Tyr Glu Pro Met Ala Cys Gly Glu Gly Gly Tyr Pro Ala Val
   250                 255                 260                 265
   TTG GAA CCG GAA GAT TGC TTA GAT ATG AAT AGG AAT CTA CCT CTA TGC     1229
   Leu Glu Pro Glu Asp Cys Leu Asp Met Asn Arg Asn Leu Pro Leu Cys
                   270                 275                 280
   CTA TCG CTT GTG GAC CGC TGT TAC AAG TCC CAT TCT GTT TTC TCT TGT     1277
   Leu Ser Leu Val Asp Arg Cys Tyr Lys Ser His Ser Val Phe Ser Cys
               285                 290                 295
   GTG TTG GCT GAC CGT TAT TGT GAA CAA CAG ATT ACT GGG GTT TAT GAG     1325
   Val Leu Ala Asp Arg Tyr Cys Glu Gln Gln Ile Thr Gly Val Tyr Glu
           300                 305                 310
   AAA TCA GGT AGG AAC CCT TAC GAT ATT AGA TCT AAG TGT GAA GCA GAG     1373
   Lys Ser Gly Arg Asn Pro Tyr Asp Ile Arg Ser Lys Cys Glu Ala Glu
       315                 320                 325
   GAT GAT TCC GGT GCC TGT TAT CAG GAA GAA ATT TAT ATC TCT GAT TAC      1421
   Asp Asp Ser Gly Ala Cys Tyr Gln Glu Glu Ile Tyr Ile Ser Asp Tyr
   330                 335                 340                 345
   TTG AAT CAG GAG GAA GTT CAA AGA GCT TTA GGG ACT GAT GTG AGT TCT      1469
   Leu Asn Gln Glu Glu Val Gln Arg Ala Leu Gly Thr Asp Val Ser Ser
                   350                 355                 360
   TTC CAA GGT TGT AGC TCG GAT GTC GGT ATC GGT TTC GCA TTC ACT GGC      1517
   Phe Gln Gly Cys Ser Ser Asp Val Gly Ile Gly Phe Ala Phe Thr Gly
               365                 370                 375
   GAT GGA CCG AGC CCA TTC CAC CAG TAC GTC GCA GAA CTT CTT GAT CAA      1565
   Asp Gly Pro Ser Pro Phe His Gln Tyr Val Ala Glu Leu Leu Asp Gln
           380                 385                 390
   GAT ATC AAT GTC TTG ATA TAT GCA GGC GAT AAG GAT TAT ATT TGT AAT      1613
   Asp Ile Asn Val Leu Ile Tyr Ala Gly Asp Lys Asp Tyr Ile Cys Asn
       395                 400                 405
   TGG CTA GGA AAT CTC GCT TGG ACT GAA AAA TTG GAA TGG AGG TAT AAC      1661
   Trp Leu Gly Asn Leu Ala Trp Thr Glu Lys Leu Glu Trp Arg Tyr Asn
   410                 415                 420                 425
   GAA GAG TAT AAA AAA CAA GTT TTG AGA ACT TGG AAG AGT GAA GAA ACA      1709
   Glu Glu Tyr Lys Lys Gln Val Leu Arg Thr Trp Lys Ser Glu Glu Thr
                   430                 435                 440
   GAT GAG ACC ATT GGC GAA ACC AAA TCT TAT GGC CCG CTA ACT TAC TTG      1757
   Asp Glu Thr Ile Gly Glu Thr Lys Ser Tyr Gly Pro Leu Thr Tyr Leu
               445                 450                 455
   AGA ATC TAT GAT GCT GGA CAC ATG GTT CCT CAC GAC CAA CCT GAA AAT      1805
   Arg Ile Tyr Asp Ala Gly His Met Val Pro His Asp Gln Pro Glu Asn
           460                 465                 470
   TCA TTA CAA ATG GTG AAT TCA TGG ATT CAG AAT ATC GCA AAG AGA TCT      1853
   Ser Leu Gln Met Val Asn Ser Trp Ile Gln Asn Ile Ala Lys Arg Ser
       475                 480                 485
   AGA ATA TAAGCATATT TCTTTACAAT TAATTTTAAA TACAAGCACC CTGAGGTATA       1909
   Arg Ile
   490
   TACTGTATGC AGTTTGTTGC ATATCTATCA TTTCTTTCGC AATTGTTCAC TTTTGATTCA    1969
   TTCTGTACAC TCTAATAAGG TTTTGCAACC TAGTAATGAT TTCCACACAT TCTTCAGCCG    2029
   ACACAGCTTC GAAATAATAT CTCCGTTCTC TATCAGGTCT GTGAACAAAA ATCTTGAAAT    2089
   ATTCTGGAAC GCGCTTAGAC CTTTTCACCA GGGTAATTTG GCTTATATGG AACGATTTCG    2149
   TCTTGACGTT TTCGTGTGTC CAATTGAAAT CACCATCAGG CCCACTTATA TAAACGTAGT    2209
   CACCATCAAT GACCAACATC CTCTCGTGTT TATTGATGAA TGACATTTGT TGTCTTCTCC    2269
   ATACCTTATA TTTATAGTAG GAACCAGCAA AGAGATCTTT GTAACTGTTA TTTCCAGAAA    2329
   CGTTATTTGA TGGTTTGGAT AAACTCCCAA GTTTAGGAGA TTTCTGGCCA TTGTTGAGAG    2389
   AAGATTTTGA GGAATTTTTG AGTTTAAAAA AGCCAGAGGT AGAGGAAGAT GTGTTATGCT    2449
   GCTTGTTGAT ACTGAATAAA TTCTTTGAGC TTGTTCTGTT CGAGGTTGGT CGAC 2503
(2) INFORMATION POUR LA SEQ ID NO: 3:
   (i) CARACTERISTIQUES DE LA SEQUENCE:
      (A) LONGUEUR: 1615 paires de bases
      (B) TYPE: acide nucléique
      (C) NOMBRE DE BRINS: double
      (D) CONFIGURATION: linéaire
   (ii) TYPE DE MOLECULE: ADNc
   (vi) ORIGINE:
      (B) SOUCHE: Kluyveromyces
   (ix) CARACTERISTIQUE ADDITIONELLE:
      (A) NOM/CLE: CDS
      (B) EMPLACEMENT: 188..1417
      (D) AUTRES RENSEIGNEMENTS: /product= "Gene de la protease A" /gene= "PRA1"
   (xi) DESCRIPTION DE LA SEQUENCE: SEQ ID NO: 3:
   ATCGATAATA GAAGTGTTGA CATAACTATA TTAAAGACAG GGTAGACGGT CAGATATATA     60
   GTAGTGTCAG TATTTTGAAC GGAGAGGAAC TTGATTAAAT CTATTATACA GTTTCCCCCA    120
   AAATTTTTCT GAAATTGTGC CGCTAACTGT TCATTAAACG GTGCTTTCTT ACAACAAAAA    180
   AATAAGC ATG CAT TTG AAT TTC CAA TCT CTT TTG CCT CTA GCT TCA TTG      229
           Met His Leu Asn Phe Gln Ser Leu Leu Pro Leu Ala Ser Leu
            1               5                  10
   TTA TTG GCT TCT TTT GAT GTT GCT GAA GCC AAG ATT CAT AAG GCC AAA      277
   Leu Leu Ala Ser Phe Asp Val Ala Glu Ala Lys Ile His Lys Ala Lys
    15                  20                  25                  30
   ATT CAA AAA CAT AAA TTG GAA GAC CAA TTG AAG GAT GTT CCA TTT GCC      325
   Ile Gln Lys His Lys Leu Glu Asp Gln Leu Lys Asp Val Pro Phe Ala
                    35                  40                  45
   GAA CAT GTG GCT CAA CTA GGT GAA AAG TAC TTA AAT AGC TTC CAA AGA      373
   Glu His Val Ala Gln Leu Gly Glu Lys Tyr Leu Asn Ser Phe Gln Arg
                50                  55                  60
   GCT TAC CCT CAA GAA TCT TTC TCT AAG GAT AAC GTT GAT GTT TTC GTT      421
   Ala Tyr Pro Gln Glu Ser Phe Ser Lys Asp Asn Val Asp Val Phe Val
            65                  70                  75
   GCC CCA GAA GGG TCT CAC AGT GTC CCA TTG ACC AAT TAC TTG AAT GCT      469
   Ala Pro Glu Gly Ser His Ser Val Pro Leu Thr Asn Tyr Leu Asn Ala
        80                  85                  90
   CAG TAT TTC ACA GAA ATT ACT TTG GGT TCG CCA CCA CAG TCT TTT AAG      517
   Gln Tyr Phe Thr Glu Ile Thr Leu Gly Ser Pro Pro Gln Ser Phe Lys
    95                 100                 105                 110
   GTT ATC TTA GAC ACT GGT TCA TCA AAC TTG TGG GTT CCA AGT GCA GAA      565
   Val Ile Leu Asp Thr Gly Ser Ser Asn Leu Trp Val Pro Ser Ala Glu
   115 120 125
   TGT GGT TCT TTG GCA TGT TTC TTG CAC ACC AAA TAT GAC CAT GAG GCT 613
   Cys Gly Ser Leu Ala Cys Phe Leu His Thr Lys Tyr Asp His Glu Ala
   130 135 140
   TCT AGC ACT TAC AAA GCT AAT GGT TCC GAG TTT GCT ATC CAA TAT GGT 661
   Ser Ser Thr Tyr Lys Ala Asn Gly Ser Glu Phe Ala Ile Gln Tyr Gly
   145 150 155
   TCT GGT TCC CTT GAA GGA TAT GTG TCT CGT GAT TTG TTG ACC ATT GGG 709
   Ser Gly Ser Leu Glu Gly Tyr Val Ser Arg Asp Leu Leu Thr Ile Gly
   160 165 170
   GAT TTA GTG ATA CCT GAC CAG GAT TTC GCT GAA GCT ACC AGC GAA CCA 757
   Asp Leu Val Ile Pro Asp Gln Asp Phe Ala Glu Ala Thr Ser Glu Pro
   175 180 185 190
   GGT TTG GCA TTT GCC TTT GGT AAA TTC GAT GGT ATT TTG GGG TTG GCT 805
   Gly Leu Ala Phe Ala Phe Gly Lys Phe Asp Gly Ile Leu Gly Leu Ala
   195 200 205
   TAC GAC TCC ATC TCT GTT AAC AGA ATC GTT CCA CCA GTG TAC AAC GCT 853
   Tyr Asp Ser Ile Ser Val Asn Arg Ile Val Pro Pro Val Tyr Asn Ala
   210 215 220
   ATC AAA AAC AAA CTT TTG GAT GAC CCA GTG TTT GCC TTT TAC TTG GGT 901
   Ile Lys Asn Lys Leu Leu Asp Asp Pro Val Phe Ala Phe Tyr Leu Gly
   225 230 235
   GAT TCT GAC AAG TCT GAA GAT GGC GGT GAA GCT TCC TTC GGT GGT ATC 949
   Asp Ser Asp Lys Ser Glu Asp Gly Gly Glu Ala Ser Phe Gly Gly Ile
   240 245 250
   GAT GAG GAG AAG TAC ACC GGT GAA ATC ACT TGG TTG CCT GTT CGT CGT 997
   Asp Glu Glu Lys Tyr Thr Gly Glu Ile Thr Trp Leu Pro Val Arg Arg
   255 260 265 270
   AAG GCT TAC TGG GAA GTC AAG TTT GAA GGT ATC GGT TTG GGT GAA GAA 1045
   Lys Ala Tyr Trp Glu Val Lys Phe Glu Gly Ile Gly Leu Gly Glu Glu
   275 280 285
   TAT GCT ACT TTA GAA GGT CAT GGT GCT GCT ATC GAC ACC GGT ACC TCT 1093
   Tyr Ala Thr Leu Glu Gly His Gly Ala Ala Ile Asp Thr Gly Thr Ser
   290 295 300
   TTG ATT GCT TTG CCA AGC GGT TTG GCT GAA ATT TTG AAC GCT GAA ATC 1141
   Leu Ile Ala Leu Pro Ser Gly Leu Ala Glu Ile Leu Asn Ala Glu Ile
   305 310 315
   GGT GCA AAG AAG GGC TGG TCT GGT CAA TAC TCC GTT GAT TGT GAA TCT 1189
   Gly Ala Lys Lys Gly Trp Ser Gly Gln Tyr Ser Val Asp Cys Glu Ser
   320 325 330
   AGA GAT AGT CTA CCA GAC TTA ACT TTG AAT TTC AAC GGT TAC AAC TTC 1237
   Arg Asp Ser Leu Pro Asp Leu Thr Leu Asn Phe Asn Gly Tyr Asn Phe
   335 340 345 350
   ACT ATT ACC GCA TAC GAT TAC ACT TTG GAA GTC TCT GGG TCT TGT ATC 1285
   Thr Ile Thr Ala Tyr Asp Tyr Thr Leu Glu Val Ser Gly Ser Cys Ile
   355 360 365
   TCT GCA TTC ACT CCA ATG GAC TTC CCA GAA CCA GTT GGT CCC TTG GCC        1333
   Ser Ala Phe Thr Pro Met Asp Phe Pro Glu Pro Val Gly Pro Leu Ala
               370                 375                 380
   ATT ATT GGT GAT GCC TTC CTA CGT AAA TAC TAC TCC ATT TAT GAT ATT        1381
   Ile Ile Gly Asp Ala Phe Leu Arg Lys Tyr Tyr Ser Ile Tyr Asp Ile
           385                 390                 395
   GGT CAT GAT GCA GTT GGT TTG GCC AAG GCT GCC TAATTGTTAA AAAAGCGATC      1434
   Gly His Asp Ala Val Gly Leu Ala Lys Ala Ala
       400                 405                 410
   GAATTGTAAC CTTTTGAATT GGAGTTCAGC TTCTATTAAC TCGACAACTC TAAAAAAATA      1494
   ATTAAATAAG ACGGTTAACT TACTGCTATA TTAATTGAAT GTCAGTTTCA CAAATCGAAT      1554
   TAGCTAACAA AGTATAACAA CACTTGGTGA CAAATAAACC TTAAAATACC TGGCAGAATT      1614
   C                                                                      1615